(19)

**Europäisches Patentamt**
**European Patent Office**
**Office européen des brevets**

(11)  **EP 1 499 289 B1**

(12)  # EUROPEAN PATENT SPECIFICATION

(45) Date of publication and mention
of the grant of the patent:
**23.07.2008 Bulletin 2008/30**

(51) Int Cl.:
***A61K 9/00*** (2006.01)

(21) Application number: **03721484.8**

(22) Date of filing: **28.03.2003**

(86) International application number:
**PCT/US2003/009487**

(87) International publication number:
**WO 2003/082207 (09.10.2003 Gazette 2003/41)**

(54) **VOLUME EFFICIENT CONTROLLED RELEASE DOSAGE FORM**

VOLUMENSPARENDE ARZNEIFORM ZUR KONTROLLIERTEN FREISETZUNG

FORME DE DOSAGE A LIBERATION CONTROLEE A VOLUME EFFICACE

(84) Designated Contracting States:
**AT BE BG CH CY CZ DE DK EE ES FI FR GB GR HU IE IT LI LU MC NL PT RO SE SI SK TR**

(30) Priority: **29.03.2002 US 368714 P**
**16.08.2002 US 404061 P**

(43) Date of publication of application:
**26.01.2005 Bulletin 2005/04**

(73) Proprietor: **ALZA CORPORATION**
**Mountain View, CA 94039-7210 (US)**

(72) Inventors:
• **WONG, Patrick, S.L.**
**Burlingame, CA 94010 (US)**

• **JAO, Francisco**
**San Jose, CA 95138 (US)**
• **EDGREN, David, E.**
**Los Altos, CA 94022 (US)**
• **SKLUZACEK, Robert, R.**
**Newark, CA 94560 (US)**
• **LI, Shu, S.L.**
**Union City, CA 94587 (US)**

(74) Representative: **Fisher, Adrian John et al**
**CARPMAELS & RANSFORD**
**43-45 Bloomsbury Square**
**London WC1A 2RA (GB)**

(56) References cited:
**WO-A-99/44591          US-A- 5 869 078**

**Description**

**BACKGROUND**

Field of the Invention: The present invention relates to a controlled-release dosage form. Specifically, the present invention relates to a controlled-release dosage form suitable for delivering soluble as well as insoluble active agents, the dosage form including an osmotic core and a bi-layer membrane system that are formulated and fabricated to allow increased drug loading efficiency for a dosage form of given dimensions.

State of the Art: Dosage forms providing the controlled release of an orally administered drug are known in the art, and the advantages of controlled release dosage forms are well appreciated in both the pharmaceutical and medical sciences. Controlled release dosage forms provide enhanced control of plasma concentrations of the administered drug over an extended period of time. Such control often enhances the therapeutic benefits of drug therapy, while reducing the possible side effects that may result from or be accentuated by large peaks or valleys in drug plasma concentration. Moreover, because controlled release dosage forms generally release the administered drug over a prolonged period of time, controlled release dosage forms tend to increase patient compliance, while reducing the number drug doses administered to a subject.

U.S. patents 3,845,770; 3,916,899; 4,008,719; 4,014,334; 4,058,122; 4,116,241; 4,160,452; 5,160,744 teach various controlled release dosage forms that allow the controlled release of a desired active agent. In general, the dosage forms disclosed in these patent references achieve the controlled release of the active agent composition through the use of a semipermeable membrane bounding an osmotic core that includes a volume of active agent composition and an expandable layer. The semipermeable membrane allows aqueous liquid to enter the osmotic core at a desired rate, thereby hydrating the expandable layer. As it hydrates, the expandable layer expands and expels the active agent included in the active agent composition. In order to achieve a desired release rate or release rate profile, the active agent composition included in a controlled release dosage may incorporate multiple formulation layers containing varying concentrations of active agent. Though known osmotic dosage forms are used to deliver active agent to a subject at a controlled rate over time, such dosage forms are generally not volume efficient, with the expandable layer typically accounting for one third or more of the weight of the osmotic core. Moreover, the fabrication of the known osmotic dosage forms can be complex and require specialized manufacturing machinery, particularly where the active agent composition must be formulated with multiple layers in order to achieve a desired release rate profile.

U.S. Patent 6,245,357 (the '357 Patent) teaches a dosage form providing controlled release of soluble or insoluble active agents. The dosage form of the '357 Patent includes two membranes forming an internal compartment including an osmotic core having a volume of an active agent composition. Advantageously, the dosage form taught in the '357 Patent does not require an expandable layer or a volume of active agent composition including multiple formulation layers in order to achieve controlled release of the active agent. However, where an expandable layer is not included in the dosage form disclosed in the '357 patent, a significant amount of residual drug may remain undelivered within the dosage form, requiring the active agent composition to include an amount of drug overage to deliver a desired dose. Though the '357 Patent discloses that an expandable layer may be included in the compartment formed by the two membranes, the '357 Patent teaches that, where included, the expandable layer again accounts for about one third or more of the weight of the osmotic core included within the compartment formed by the two membranes. Therefore, despite its benefits, the controlled release dosage form disclosed in the '357 Patent also exhibits shortcomings that reduce the amount of active agent that can be loaded in and delivered from the dosage form

Dosage forms formulated at low drug loading efficiency can be bulky and so large that patients in need are unwilling or unable to swallow them. Providing oral dosage forms of an acceptable size is a particularly difficult challenge where the dose of active agent is high and the aqueous solubility of the active agent is low. Moreover, the rate controlling membranes of such dosage forms are typically applied in coating equipment having a fixed volume per unit batch of dosage forms coated, and as the size of the dosage form coated decreases, the number of dosage forms that can be coated per unit batch increases, leading to an increase in process throughput. Significantly, providing a dosage form with relatively higher drug loading efficiency allows a desired does of active agent to be delivered to a subject using a relatively smaller dosage form, and as the size of the dosage form decreases, the ease with which the dosage form can be administered increases, while the cost of manufacturing the dosage form decreases. Therefore, it would be an improvement in the art to provide a controlled release dosage form that is easily manufactured, produces a desired release rate or release rate profile for a desired soluble or insoluble active agent, and provides increased loading efficiency of soluble or insoluble active agents.

SUMMARY OF THE INVENTION

The present invention includes a dosage form that facilitates the controlled release of an active agent at a desired release rate or release rate profile. In each embodiment, the dosage form of the present invention includes a bi-layer membrane system and an osmotic core. The bi-layer membrane system includes a semipermeable membrane and an osmosensitive membrane and forms an internal compartment that is occupied by the osmotic core. The osmotic core of the dosage form of the present invention is formulated with osmotic activity and includes and active agent composition and a light push layer. The dosage form of the present invention additionally includes a passageway that is formed through the bi-layer membrane system and permits expulsion of the active agent composition from the dosage form during operation. Preferably, the dosage form of the present invention is manufactured by first forming the osmotic core and then coating the osmotic core with the bi-layer membrane system. If desired, the dosage form of the present invention may further be provided with one or more membranes or layers exterior to the bi-layer membrane system.

The bi-layer membrane system and the osmotic core of the dosage form of the present invention are formulated and formed to provide controlled release of the active agent included in the active agent composition of the osmotic core. Moreover, the construction and formulation the dosage form of the present invention allows increased loading of active agent composition within the osmotic core of the dosage form (*i.e.,* the active agent composition may account for about 75%, or more, of the total weight of the osmotic core), while reducing, or eliminating, the need to include an overage of active agent within the dosage form in order to ensure delivery of a desired dose. Advantageously, such characteristics of the dosage form of the present invention allow a given dose of a desired active agent to be delivered in a controlled manner using a device of relatively smaller dimensions.

BRIEF DESCRIPTION OF THE DRAWINGS

FIG. 1 and FIG. 2 provide schematic illustrations in cross section of two different embodiments of the dosage form of the present invention. Because the various features illustrated in FIG. 1 and FIG. 2 are provided for illustrative purposes only, they are not necessarily drawn to scale and are not meant to exactly represent the features included in a dosage form according to the present invention.

FIG. 3 provides a schematic illustration of a dosage form according to the present invention during operation and delivery of an active agent. Because the various features illustrated in FIG. 3 are provided for illustrative purposes only, they are not necessarily drawn to scale and are not meant to exactly represent the features included in a dosage form according to the present invention.

FIG. 4 and FIG. 5 provide graphs illustrating the permeability of exemplary osmosensitive membranes as a function of osmotic pressure.

FIG. 6 provides a graph illustrating the weight gain of an exemplary osmosensitive membrane component as a function of osmotic pressure and time immersed in an osmoagent.

FIG. 7 provides a graph illustrating the weight gain of an exemplary osmosensitive membrane component as a function of osmotic pressure and time immersed in a different osmoagent.

FIG. 8 provides a 3-dimensional surface plot and a surface plot equation illustrating the permeability of an exemplary osmosensitive membrane as a function of osmotic pressure and time in the presence of the osmoagent, sodium chloride, and illustrating the corresponding surface plot equation defining the permeability response.

FIG. 9 provides a 3-dimensional surface plot and a surface plot equation illustrating the permeability of an exemplary semipermeable membrane as a function of osmotic pressure and time in the presence of the osmoagent, sodium chloride, and illustrating the corresponding surface plot equation defining the permeability response.

FIG. 10 provides a graph illustrating the release rate profile and delivery efficiency of nifedipine achieved by a first exemplary dosage form according to the present invention.

FIG. 11 depicts a graph, which illustrates the release rate profile and delivery efficiency of nifedipine as achieved by a first experimental control dosage form that does not embody all the features of a dosage form of the present invention.

FIG. 12 provides a graph illustrating the release rate profile and delivery efficiency of nifedipine achieved by a second exemplary dosage form according to the present invention

FIG. 13 depicts a graph, which illustrates the release rate profile and delivery efficiency of nifedipine achieved by a second experimental control dosage form that does not embody all the features of a dosage form of the present invention.

DETAILED DESCRIPTION OF THE INVENTION

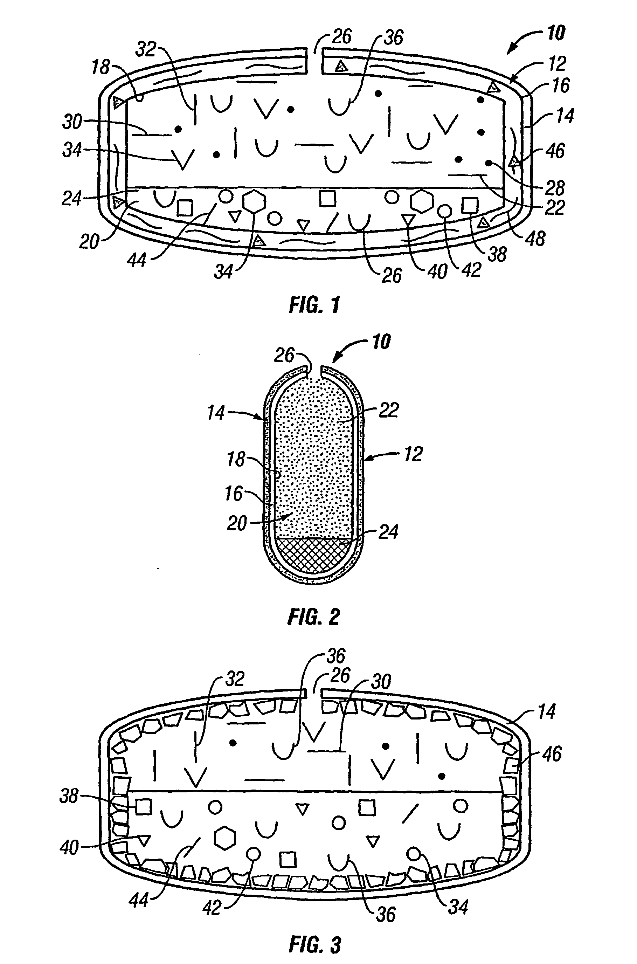

An exemplary dosage form 10 according to the present invention is illustrated in FIG. 1. As represented by the dosage form 10 illustrated in FIG. 1, the dosage form 10 of the present invention includes a bi-layer membrane system 12 including an outer semipermeable membrane 14 and an inner osmosensitive membrane 16. The bi-layer membrane system 12 of the dosage form 10 of the present invention forms an internal compartment 18 that is occupied by an osmotic core 20 that includes an active agent composition 22 and a light push layer 24. The dosage form 10 of the present invention also includes a delivery passageway 26 through which the active agent 28 included in the active agent composition 22 is delivered during operation of the dosage form 10. Both the bi-layer membrane system 12 and the osmotic core 20 of the dosage form 10 of the present invention are formulated to function in concert and fabricated to facilitate controlled delivery of the active agent 28 included in the active agent composition 22, while allowing increased loading efficiency of the active agent composition 22 within the internal compartment 18 formed by the bi-layer membrane system 12.

The active agent composition 22 included in the osmotic core 20 of the dosage form 10 of the present invention may include any desired active agent 28. The term "active agent," as it is used herein, includes medicines or active therapeutic compounds, therapeutic proteins, therapeutic peptides, nutrients, vitamins, food supplements, and any other beneficial agents that provide a therapeutic benefit to animals, including humans, farm animals, and zoo animals. The active agent 28 may be soluble or insoluble. As used herein, the term "soluble" is used to characterize materials that, when added to water thermostated at 37° C, dissolve and form a saturated concentration that is at least 20 milligrams per milliliter, while the term "insoluble" refers to those compounds that, when added to water thermostated at 37° C, dissolve to form a saturated concentration that is less than 20 milligrams per milliliter. "Saturated concentration" refers to that concentration within the aqueous phase of the solution that is in dynamic equilibrium with undissolved solid solute present in the mixture such that the net mass of solute dissolved in the aqueous phase is constant with time. Regardless of the active agent 28 included in the active agent composition 22, however, the active agent composition 22 included in the osmotic core 20 of the dosage form 10 of the present invention will preferably account for three fourths, or more, of the weight of the osmotic core 20 occupying the internal compartment 18.

The active agent composition 22 is formulated as an initially solid or semisolid material that may be manipulated or formed during the manufacture of the osmotic core 20 of the dosage form 10 of the present invention. However, the various constituents of the active agent composition 22 are also formulated such that, as the active agent composition 22 absorbs an aqueous fluid from the environment of operation (e.g., aqueous gastrointestinal fluid) through the bi-layer membrane system 12, the active agent composition 22 is converted into a solution, a liquid, a gel, or a gel-like substance that can be expelled through the delivery passageway 26 provided.

Representative active agents that may be included in the active agent composition 22 include, for example, immunosuppressive and immunoreactive agents, antiviral and antifungal agents, antineoplastic agents, analgesic and anti-inflammatory agents, antibiotics, anti-epileptics, anesthetics, hypnotics, sedatives, anti sychotic agents, neuroleptic agents, antidepressants, anxiolytics, anticonvulsant agents, antagonists, neuron blocking agents, anticholinergic agents and cholinomimetic agents, antimuscaraicic and mucarinic agents, antiadrenergic and antiarrythmics, antihypertensive agents, hormones, and nutrients. A detailed description of these and other active agents that may be included in the active agent composition 22 of the dosage form 10 of the present invention is found in Remington's Pharmaceutical Sciences, 18th editions, 1990, Mack Publishing Co., Philadelphia, PA.

Specific examples of soluble active agents that may be delivered using the dosage form 10 of the present invention include, for example, acebutolol, acetazolamide, acetophenazine, acetylcarnatine, acyclovir, albumin, albuterol, amantadine, ambenoium, amiloride, amitriptyline, amoxicillin, ampetameine, ampicillin, anisotropine, arecoline, atenolol, atracurium, atropine, azatadine, bacitracin, benzsepril, benzphetamine, benztropine, beraprost, betamethasone, betaxolol, bleomycine, brompheniramine, buprenorphine, bupropion, buspirone, calcitonin, captopril, carbinoxamine, carboplatin, cefadroxil, cefazolin, cefixime, cefotaxime, cefotetan, cefotixin, deftriaxone, cefuroxime, chlordiazepoxide, chlorpheniramine, chlorpromazine, ciclopirox, cilastatin, cietidine, clidinium, clindamycin, clomipramine, clonidine, clorazepate, codeine, cromolyn, cyclobenzaprine, deprenyl, desipramine, desmopressin, dexamethasone, dezocine, diclofenac, dicyclomine, diethylpropion, diltiazem, diphenhydramine, dipivefrin, disopyramide, dopamine, dotheipin, doxepin, doxorubicin, doxycycline, encainide, ephedrine, epinephrine, epoetin-alpha, ergonovine, erythromycin, estradiol, conjugated estrogens, esterified estrogens, fenfluramine, fentanyl, fluoxetine, fluphenazine, flurazepam, gepirone, glycopyrrolate, granisetron, guaifenesin, guanadrel, guanethidine, hexobendine, hexoprenaline, histidine, homatropine, hydralzine, hydrocodone, hydrocortisone, hydroxychloroquine, hydroxyzine, hyoscyamine, imipramine, indomethacin, ipratropium bromide, isoproterenol, isosorbide, ketorolac, leuprolide, levobunolol, levorphanol, lidocaine, lisinopril, lithium, mecamylamine, mefenamic acid, menotropins, meperidine, mephentermine, metaproterenol, methamphetamine, methdilazine, methimazole, methotrimeprazine, methscopolamine, methylphenidate, methylprednisolone, metoprolol, metrifonate, metronidazole, mexiletine, midazolam, minocycline, molidone, morphine, moveltipril, nalbuphine, naloxone, naltrexone, naproxen, neostigmine, netilmicin, nicorandil, nitrofuranatoin, norfenefrine, oslalazine, ondansetron, oxybutynin, oxycodone, oxymorphone, oxytetracycline, pamidronate, pancopride, parathyroid hormone, penicillin G, pentostatin, pentoxifylline, phenelzine, phenmetrazine, phenobarbital, phenoxybenzamine, phentermine, phenylephrine, pilocarpine, pravastatin, probarbital, prochlorperazine, procyclidine, promethazine, propantheline, propiomazine, propranolol, protryptyline, psuedoephedrine, pyridostigmine, quinapril, quinidine, ramoplanin, ranitidine, rilmenidine, ritodrine, saralasin, scopolamine, sulfadiazine, tacrine, teicoplanin, terazosin, terbutaline, tertatolol, tetracaine, tetracycline, theophylline, thiethylperazine, thioridazine, thiothixene, ticlopidine, timolol, tobramycin, tolmetin, tranylcypromine, trapidil, trifluoperazine, trimeprazine, trimetazidine, trimethobenzamide, triprolidine, tubocurarine, valproic acid, vancomycin, verapamil, warfarin, zidovudine, and soluble derivatives, pro-drugs, isomers, and salts of the above.

Specific examples of insoluble active agents that may be delivered using the dosage form 10 of the present invention include, for example, acenocoumarol, acetaminophen, acetazolaminde, acetophenazine, acyclovir, albuterol, allopurinol, aprazolam, alteplase, amantidine, aminopyrine, amiloride, amiodarone, amitriptyline, amlodipine, amoxapine, amoxicillin, amphotericin B, ampicillin, apomorphine, aspirin, astemizole, atenolol, atracurium, atropine, auranofin, azathioprine, aztreonam, bacitracin, baclofen, beclomethasone, benazepril, bendroflumethiazide, betamethasone, biperiden, bitolterol, bromocriptine, buclizine, bumetanide, buprenorphine, busulfan, butorphanol, cadralazine, calcitriol, carbamazepine, carbidopa, carboplatin, cefaclor, cefazolin, cefoxitin, ceftazidime, cephalexin, chloramphenicol, chlordiazepoxide, chlorpheniramine, chlorpromazine, chlorpropamide, chlorthalidone, chlorzoxazone, cholestyramine, cimetidine, ciprofloxacin, cisapride, cisplatin, clarithromycin, clemastine, clonazepam, clotrimazole, clozapine, codeine, cyclizine, cyclobarbital, cyclosporine, cytarabine, chlorothiazide, cyclophosphamide, dacarbazine, deflazacort, deserpidine, desanoside, desogestrel, desoximetasone, dexamethasone, dextromethorphan, dezocine, diazepam, diclofenac, dicyclomine, diflunisal, digitoxin, digoxin, dihydroergotamine, dimenhydrinate, diphenoxylate, dipyridamole, disopyramide, dobutamine, domperidone, dopexamine, doxazosin, doxorubicin, doxycycline, droperidol, enalapril, enoximone, ephedrine, epinephrine, ergotoloids, ergovine, erythromycin, estazolam, estradiol,ethinyl estradiol,etodolac, etoposide, famotidine, felodipine, fenfluramine, fenoprofen, fentanyl, filgrastim, finasteride, fluconazole, fludrocortisone, flumazenil, flunisolide, fluocinonide, fluorourcil, fluoxetine, fluoxymesterone, fluphenazine, fluphenazine, flurbiprofen, flutamide, fluticasone, furosemide, ganciclovir, gemfibrizil, glipizide, glyburide, gramicidin, granisetron, guaifenesin, guanabenz, guanadrel, guanfacine, haloperidol, heparin, homatropine, hydralazine, hydrochlorothiazide, hydrocodone, hydrocortisone, hydromorphone, hydroxyzine, hyoscyamine, ibudilast, ibuprofen, isosorbide dinitrate, pseudoephedrine, cholchicine, secoverine, progesterone, naloxone, imipramine, indapamide, indomethacin, insulin, ipratropium, isocarboxazid, isopropamide, isosorbide,isotretinoin, isradipine, itraconazole, ketoconazole, ketoprofen, levonorgestrel, levorphanol, lidocaine, lindane, liothyronine, lisinopril, lithium, lomefloxacin, loperamide, loratadine, lorazepam, lovastatin, loxapine, mabuterol, maprotiline, mazindol, meclizine, medroxyprogesteron, mefenamic acid, melatonin, meperidine, mephentermine, mesalazine, mestranol, methdilazine, methotrimeprazine, methotrexate, methoxsalen, methoxypsoralen, methyclothiazide, methylphenidate, methylprednisolone, methyltestosterone, methysergide, metocurine iodide,metolazone, metronidazole, miconazole, midazolam, milrinone, minocycline, minoxidil, mitomycin, molsidomine, mometasone, morphine, mupirocin, muroctasin, nabumetone, nadolol, naltrexone, neostigmine, nicardipine, nicorandil, nicotine, nifedipine, nimodipine, nitrendipine, nitrofurantoin, nitroglycerin, norfloxacin, nystatin, octreotide, ofloxacin, omeprazole, oxaprozin, oxazepam, oxycodone, oxyphencyclimine, oxytetracycline, paclitaxel, paramethasone, paroxetine, pemoline, penicillin, pentaerythritol, pentamidine, pentazocine, pergolide, perphenazine, phenazopyridine, phenelzine, phenobarbitol, phenoxybenzamine, phenytoin, physostigmine, pimozide, pindolol, polythizide, prazepam, prazosin, prednisolone, prednisone, probucol, prochloperazine, procyclidine, propofol, propranolol, propylthiouracil, pyrimethamine, quinidine, ramipril, rescinnamine, reserpine, rifabutin, rifapentine, respiridone, salmeterol, sertraline, siagoside, simvastatin, spironolactone, sucralfate, sulfadiazine, sulfamethoxazole, sulfamethizole, sulindac, sulpiride, tamoxifen, tandospirone, temazepam, terazosin, terbinafine, terconazole, terfenadine, tetracaine, tetracycline, theophylline, thiethylperazine, thioridazine, thiothixene, thyroxine, timolol, topiramate, tranylcypromine, trazodone, tretinoin, triamcinolone, trimethoprim, triazolam, trichlormethiazide, trihexphenidyl, trioxsalen, vinblastine, vitamin B, and insoluble derivatives, pro-drugs , isomers, and salts of the above.

In order to provide an active agent composition 22 that converts to a solution, a liquid, a gel, or a gel-like substance upon the absorption of an aqueous fluid, the active agent composition 22 optionally includes a pharmaceutically acceptable hydrogel 30 (represented by horizontal dashes in FIG. 1). The hydrogel 30 included in the active agent composition 22 not only facilitates the creation of an active agent composition 22 that converts to a solution, a liquid, a gel, or a gel-like substance as the active agent composition 22 absorbs aqueous fluid, but the hydrogel 30 included in the active agent composition 22 also creates an osmotic pressure gradient across the bi-layer membrane system 12, causing aqueous fluid from the environment of operation to be absorbed into the active agent composition 22 through the bi-layer membrane system 12 of the dosage form 10.

Numerous different polymer hydrogels are suitable for use in the active agent composition 22 of the dosage form 10 of the present invention. Exemplary polymer hydrogels include the following: a maltodextrin polymer comprising the formula $(C_6H_{10}O_5)_\lambda \bullet H_2O$, wherein $\lambda$ is 3 to 7,500, and the maltodextrin polymer possesses a 500 to 1,250,000 grams per mole number-average molecular weight; a poly(alkylene oxide), such as, a poly(ethylene oxide) or a poly(propylene oxide) having a 7,000 to 750,000 number-average molecular weight, or, more specifically, a poly(ethylene oxide) having at least one of a 100,000, a 200,000, a 300,000, or a 400,000 number-average molecular weight; an alkali carboxyalkylcellulose having a 10,000 to a 175,000 number-average molecular weight, wherein the alkali is sodium, lithium, potassium, or calcium, and the alkyl is 1 to 5 carbons, such as a methyl, ethyl, propyl, or butyl group; or a copolymer of ethylene-acrylic acid, such as, for example, methacrylic or ethacrylic acid having a 10,000 to 1,500,000 number-average molecular weight. Non-polymeric compounds, such as monosacharrides and disacharrides, are also suitable for use as hydrogel compounds in the active agent composition 22. Though the precise amount of polymer hydrogel 30 included in the active agent composition 22 will vary depending upon, among other factors, the desired viscosity of the active agent composition 22 during operation, the type of active agent 28 to be delivered, and the magnitude of osmotic pressure gradient desired across the bi-layer membrane system 12, where the active agent composition 22 includes a hydrogel 30, the amount of hydrogel 30 included will preferably range between about 5 mg and 400 mg. Moreover, though the active agent composition 22 may be formulated using a single type of hydrogel material, more than one different type of hydrogel, including blends of different molecular weight polymer hydrogels of the same type, may also be used in the active agent composition 22.

The active agent composition 22 may also include a binder 32 (represented in FIG. 1 by vertical dashes). The binder 32 imparts cohesive qualities to the active agent composition 22 and may be provided in a solution form or a dry form to prepare the active agent composition 22. Binders that may be included in the active agent composition 22 include, for example, starch, gelatin, molasses, methylcellulose, hydroxypropylcellulose, hydroxypropyl methylcellulose, and a vinyl polymer exhibiting a 5,000 to 350,000 number-average molecular weight, such as a poly-n-vinylamide, poly-n-vinylacetamide, poly(vinyl pyrrolidone) (also known as poly-n-vinylpyrrolidone), poly-n-vinylcaprolactone, poly-n-vinyl-5-methyl-2-pyrrolidone, or poly-n-vinylpyrrolidone copolymers with a member selected from, for example, vinyl acetate, vinyl alcohol, vinyl chloride, vinyl fluoride, vinyl butyrate, vinyl laureate, and vinyl stearate. If desired, the active agent composition 22 may include more than one different type of binder 32. Where one or more binders are included in the active agent composition 22, the binder 32 or mixture of binders 32 may represent up to about 100 mg of the active agent composition 22, and preferably between about 0.01 mg and 50 mg.

A tableting lubricant 34 (represented in FIG. 1 by the letter "v") may also be included in the active agent composition 22. The tableting lubricant 34 lessens adhesion of the active agent composition 22 to tooling, such as die walls or punch faces, or machinery used during manufacture of the active agent composition 22. Tableting lubricants suitable for use in the active agent composition 22 of the dosage form 10 of the present invention include, for example, polyethylene glycol, sodium stearate, oleic acid, potassium oleate, caprylic acid, sodium stearyl fumarate, magnesium palmitate, calcium stearate, zinc stearate, magnesium stearate, magnesium oleate, calcium palmitate, sodium sebacate, potassium laureate, stearic acid, salts of fatty acids, salts of alicyclic acids, salts of aromatic acids, oleic acid, palmitic acid, a mixture of a salt of a fatty, alicyclic, or aromatic acid, and a mixture of magnesium stearate and stearic acid. If included in the active agent composition 22 of the present invention, the tableting lubricant 34 preferably accounts for between about 0.01 mg and 20 mg of the active agent composition 22.

In order to achieve a desired osmotic pressure gradient across the bi-layer membrane system 12, the active agent composition 22 may also include an osmoagent 36 (also known as an osmotically effective compound or an osmotically effective solute). Like the polymer hydrogel 30, the osmoagent 36 (represented in FIG. 1 as "u" shaped lines) creates an osmotic pressure gradient across the bi-layer membrane system 12, causing aqueous environmental fluid to be taken into the osmotic core material (i.e., the active agent composition 22 and light push layer 24) occupying the internal compartment 18. Any compound capable of generating an osmotic pressure gradient across the bi-layer membrane system 12 while not adversely affecting the performance or function of the bi-layer membrane system 12, the active agent composition 22, or the light push layer 24 may be used as an osmoagent 36 in the active agent composition 22. Examples of osmoagents that may be formulated into the active agent composition 22 include adipic acid, alanine, ammonium phosphate dibasic, arginine, ascorbic acid, boric acid, calcium gluconate, calcium nitrate, citric acid, dextrose, diammonium succinate, disodium adipate, dipotassium adipate, dipotassium succinate, disodium succinate, fructose, fumaric acid, galactose, gluconodeltalactone, glutaric acid, glycine, lactose, lysine, magnesium benzoate, magnesium sulfate, malic acid, maleic acid, mannitol, monosodium glutamate, monopotassium adipate, monosodium adipate, monopotassium succinate, monosodium succinate, potassium bicarbonate, potassium chloride, potassium citrate, potassium phosphate dibasic, potassium phosphate monobasic, dipotassium succinate, potassium sodium bitartrate, potassium sulfate, sodium ascorbate, sodium bicarbonate, sodium carbonate, sodium chloride, sodium citrate, sodium fumarate, sodium nitrite, sodium glycerophosphate, sodium glycinate, sodium potassium tartrate, sodium EDTA, sodium phosphate dibasic, sodium phosphate monobasic, disodium succinate, sodium phosphate, sodium tartrate, sodium bisulfate, sodium bitartrate, sorbitol, succinic acid, sucrose, sucrose acetate isobutyrate, tartaric acid, urea, xylose, xylitol, and blends of two or more selected from the group of these osmoagents. Where included in the active agent composition 22, the precise amount of osmoagent 36 will vary depending on, among other factors, the materials used in both the active agent composition 22 and the light push layer 24, the amount and type of active agent 28 to be delivered, the desired release rate of the active agent 28, and that nature of the osmosensitive membrane 16 included in the bi-layer membrane system 12.

The light push layer 24 included in the osmotic core 20 of the dosage form 10 of the present invention is formulated both to create an osmotic pressure gradient across the bi-layer membrane system 12 and to expand as water is imbibed into the internal compartment 18 from the environment of use. The light push layer 24 included in the dosage form 10 of the present invention works to expel hydrated active agent composition 22 from the dosage form 10 as the material included in the light push layer 24 expands. However, as its name implies, the light push layer 24 included in the osmotic core 20 of the dosage form 10 of the present invention includes relatively less material than previous push layers and, therefore, accounts for less of the total weight of the osmotic core 20. Instead of accounting for one third or more of the total weight of the osmotic core 20, as is generally necessary in previous dosage forms including a push-type expandable layer, the light push layer 24 of the dosage form 10 of the present invention accounts for less than one third, preferably about one fourth or less, of the weight of the total weight of the osmotic core 20, allowing a relatively higher proportion of the osmotic core 20 to be formed by the active agent composition 22. The light push layer 24, therefore, facilitates an increase in the active agent loading efficiency of a dosage form of a give size or weight. Moreover, we have surprisingly and unexpectedly found that by pairing the bi-layer membrane system 12 as described herein with a light push layer 24 of appropriate osmotic activity, the dosage form 10 of the present invention may be fabricated to release a desired active agent at a controlled rate over a prolonged period of time while additionally providing highly efficient active agent delivery.

The relative weight of the light push layer 24 included in the osmotic core 20 of the dosage form 10 of the present invention will depend, at least in part, on the shape or relative proportions of the osmotic core 20 itself. Generally, the osmotic core 20 will be formed as a compressed tablet, and the overall relative shape or relative proportions of a compressed tablet are often characterized by the ratio of the width of the tablet to the height of the tablet, or the "aspect ratio" of the tablet. The height dimension is measured as the distance between the two surfaces formed by the upper and lower punch tips that come together in the operation used to form the tablet. The width dimension is pre-set by the fixed dimension of the die cavity in which the tablet is compressed. The aspect ratio of a tablet shaped generally as depicted in FIG. 1, for example, would be 1.0 or greater, most typically about 1.2 to 2.0, while the aspect ratio of a tablet shaped generally as depicted in FIG. 2 (a "longitundinally compressed tablet" or "LCT") would be less than 1.0, most typically about 0.4 to 0.5. In general, where the dosage form 10 of the present invention is designed as a compressed tablet having an aspect ratio of about 1.0 or greater, the light push layer 24 will initially account for about one-fourth of the weight of the osmotic core 20 of the dosage form 10. However, as manufacturing technology improves, the light push layer 24 of an osmotic core 20 having an aspect ratio of about 1.0 or greater may account for even less of the total weight of the osmotic core 20. This is because, presently, where the osmotic core 20 is manufactured as a compressed tablet with an aspect ratio of about 1.0 or greater, the minimum weight of the light push layer 24 is dictated by limitations in known press layering processes, not by performance considerations. Therefore, as manufacturing processes permit, osmotic cores suitable for use in the dosage form 10 of the present invention may be manufactured to include a light push layer 24 that accounts for significantly less than one-fourth of the total weight of the osmotic core 20, while still being manufactured as a compressed tablet having an aspect ratio of about 1.0 or greater.

Though the minimum weight of a light push layer 24 included in a compressed tablet with an aspect ratio of about 1.0 or greater is presently limited to about one-fourth of the total weight of the osmotic core 20, where the dosage form 10 of the present invention is designed as a longitudinally compressed tablet (shown in FIG. 2), the dosage form 10 may be include a light push layer 24 that accounts for only about one quarter to one fifth, or less, of the weight of the osmotic core 20. Due to the relatively smaller diameter to volume ratio of material layers included in a compressed tablet having an aspect ratio less than 1.0, a layer of material of a given volume in a longitudinally compressed tablet will generally be thicker and, therefore, easier to fabricate using state of the art press-layering processes than in a tablet compressed with an aspect ratio of about 1.0 or greater. Therefore, where the osmotic core 20 of dosage form 10 of the present invention is designed as a compressed tablet with a small aspect ratio, the dosage form 10 of the present invention provides an even greater increase in the amount of active agent composition 22 that can be loaded in and delivered from a device of given dimensions. While FIG. 1 and FIG. 2 generally refer tablets formed with round dies, osmotic core 20 of the present invention can be fabricated with both large and small aspect ratios and with die shapes other than round including oval, triangular, square, banana-shaped, kidney-shaped, polygonal such as pentagon or hexagon, and the like.

The light push layer 24 included in the dosage form 10 of the present invention also imparts other demonstrable advantages. For example, the light push layer 24 included in the dosage form 10 of the present invention increases the efficiency with which the dosage form 10 of the present invention delivers a chosen active agent 28 relative to either an osmotic dosage form that lacks any sort of push layer or an osmotic dosage form that includes a heavier push layer. Osmotic dosage forms that do not include any sort of push layer generally retain an amount of residual active agent within the dosage form, even after the dosage form has stopped functioning. However, as it expands, the light push layer 24 backfills the internal compartment 18 of the dosage form 10 of the present invention and, in effect, sweeps the internal compartment 18 clean of residual active agent 28, thereby providing increased delivery efficiency relative to dosage forms lacking any sort of push layer. In addition, osmotic dosage forms that include a heavier push layer tend to exhibit delivery inefficiencies, as the expandable material forming the push layer mixes with or channels through the active agent formulation. This interlayer mixing is caused, at least in part, by a mismatch between the rate of swelling of the expandable material of push layer and the rate of hydration of the active agent composition. To compensate for this mismatch, previous systems required the use of a heavier push layer consisting of more expandable material in order to sustain continuous, non-accelerating push layer expansion over prolonged periods of active agent delivery. Where an osmotic dosage form includes a heavier push layer, the heavier push layer causes mixing of the active agent composition and the push layer where the two layers interface, and active agent material that mixes into the push layer is effectively trapped inside the dosage form and rendered undeliverable. In contrast to previous, heavier push layers, the light push layer 24 included in the dosage form 10 of the present invention hydrates at accelerating rate with time, thereby substantially reducing or eliminating any mixing of the active agent composition 22 with the light push layer 24. This reduction in mixing of the active agent composition 22 with the light push layer 24 increases the delivery efficiency of active agent 28 achieved by the dosage form 10 of the present invention.

Advantageously, the increased delivery efficiency provided by the dosage form of the present invention further enhances the amount of active agent that can be delivered using a dosage form of given dimensions. Due to the delivery inefficiencies of previous controlled-release osmotic dosage forms, it was generally necessary to include an overage, or excess, of active agent composition, typically a 10% overage, in order to achieve delivery of a desired dose of active agent. However, the delivery efficiency of the dosage form 10 of the present invention reduces or eliminates the need to include any overage of active agent 28, allowing a given dose of active agent 28 to be delivered using a relatively smaller and less expensive dosage form 10. Therefore, the dosage form 10 of the present invention not only allows increased loading of active agent composition 22 into a dosage form 10 of given dimensions, but the increased delivery efficiency provided by the dosage form 10 of the present invention enables the dosage form of the present invention to deliver more of the active agent loaded therein, which enables a further decrease in the size of the dosage form required to deliver a desired dose of active agent.

To provide a layer of material that both creates an osmotic pressure gradient and expands as it absorbs water, the light push layer 24 preferably includes an osmopolymer 38 (represented by squares in FIG. 1). The osmopolymer 38 material included in the light push layer 24 typically possesses a higher molecular weight than the hydrogel 30 included in the active agent composition 22. Moreover, the osmopolymer 38 material included in the light push layer 24 not only expands as it imbibes aqueous fluid, but also serves to generate an osmotic pressure gradient across the bi-layer membrane system 12 of the dosage form 10 of the present invention. Preferably, the light push layer 24 is manufactured using between about 10 mg and about 400 mg of osmopolymer 38.

Any suitable osmopolymer may be used in the light push layer 24 of the dosage form 10 of the present invention. However the osmopolymer 38 included in the light push layer 24 is preferably selected from a polyalkylene oxide, a carboxyalkylcellulose, or a polyacrylate material. Representative polyalkylene oxides that may be used in the light push layer 24 include, for example, polyalkylene oxides possessing number-average molecular weights ranging between about 1,000,000 and 10,000,000 grams per mole, polymethylene oxide, polyethylene oxide, polypropylene oxide, polyethylene oxide having a 1,000,000 number-average molecular weight, polyethylene oxide possessing a 2,000,000 number-average molecular weight, polyethylene oxide comprising a 3,000,000 to 8,000,000 number-average molecular weight, cross-linked polymethylene oxide possessing a 1,000,000 number-average molecular weight, and polypropylene oxide of 1,200,000 number-average molecular weight. Typical carboxyalkylcellulose materials for use in the light push layer 24 include, for example, carboxyalkylcellulose materials possessing a 200,000 to 7,250,000 number-average molecular weight. Specific carboxyalkylcellulose materials for use in the light push layer 24 include members selected from the group including sodium carboxymethyl cellulose having a degree of substitution (DS) of 0.38-0.55, a sodium carboxymethyl cellulose having a DS of 0.66-0.90, a sodium carboxymethyl cellulose having a DS of 0.80-0.95, and a sodium carboxymethyl cellulose having a DS of at least 1.20. As used herein, the term "degree of substitution" indicates the average number of hydroxyl groups originally present on the anyhydroglucose unit comprising the cellulose polymer that are replaced by a substituting group.

An especially preferred sodium carboxymethyl cellulose polymer for us in the light push layer 24 is characterized by DS of 0.66-0.90 and a number-average molecular weight of 700,000. Other carboxymethyl celluloses for this application include members selected for the group of calcium carboxymethyl cellulose, magnesium carboxymethyl cellulose, and potassium carboxymethyl celluloses. Other exemplary polymers include, sodium carboxymethyl starch, alginates such as sodium alginate, natural gums such as agar, gum arabic, gum karaya, locus bean gum, gum tragacanth, gum ghatti, guar gum, xanthan gum, gelatin, pre-gelatinized starch, carrageenan, acrylates such as linear polyacrylic acid, crosslinked polyacrylic acid, sodium polyacrylic acid, and potassium polyacrylic acid. Though the light push layer 24 may be formulated using a single osmopolymer material, more than one different type of osmopolymer or blends of the same osmopolymers having different molecular weights may be incorporated into the light push layer 24.

The light push layer 24 may also include osmoagent 36 in order to achieve a desired osmotic pressure gradient across the bi-layer membrane system 12. The concentration of osmoagent 30 in the light push layer 24 is typically less than the concentration of osmoagents in previous, heavier push layers, which are typically formulated with about 20 wt% to about 30 wt% of osmoagent. In contrast, the light push layer 24 of a dosage form 10 of the present invention is typically formulated with less than 20 wt% osmoagent, and preferably with less than 15 wt% osmoagent. The type of osmoagent in the light push layer 24 may be the same or different than the type of osmoagent included in the active agent composition 22. Any compound capable of generating an osmotic pressure gradient across the bi-layer membrane system 12 without adversely affecting the performance or function of the bi-layer membrane system 12, the active agent composition 22, or the light push layer 24 may be used in the light push layer 24. Examples of osmoagents that may be used in the active agent composition 22 of the dosage form 10 of the present invention include, for example, osmotic salts, such as sodium chloride, potassium chloride, magnesium sulfate, lithium phosphate, lithium chloride, sodium phosphate, potassium sulfate, sodium sulfate, and potassium phosphate, osmotic carbohydrates, such as glucose, mannitol, maltose, fructose, maltose and sorbitol, urea, osmotic acids, such as tartaric acid, citric acid, succinic acid, malic acid, maleic acid, and potassium acid phosphate, and mixtures of osmoagents such as sodium chloride and urea. Presently, where an osmoagent is incorporated into the light push layer 24, the light push layer preferably includes up to about 200 mg of osmoagent 36. Even more preferably, the light push layer 24 includes between about 0.5 and 75 mg of osmoagent 36. However, the precise amount of osmoagent included in the light push layer 24 will vary depending on, among other factors, the materials used in both the active agent composition 22 and the light push layer 24, the amount of active agent 28 to be delivered, the desired release rate of the active agent 28, and that nature of the osmosensitive membrane 16 included in the bi-layer membrane system 12.

The light push layer 24 may also include a suspending agent 40 to provide stability and homogeneity to the light push layer 24. The suspending agent 40 (represented by clear triangles in FIG. 1) may include, for example, a hydroxypropyl alkylcellulose having a cellulose chain that is straight or branched, an alkyl of 1 to 7 carbons, and a 9,000 to 450,000 number-average molecular weight. The hydroxypropyl alkylcellulose may be represented by hydroxypropyl methylcellulose, hydroxypropyl ethylcellulose, or hydroxypropyl butylcellulose. Other cellulose derivatives that can serve as suspending agents include such celluloses as hydroxy ethylcellulose, hydroxypropyl cellulose, and hydroxy butylcellulose. Preferably, where the light push layer 24 includes a suspending agent 40, the suspending agent 40 accounts for up to about 75 mg of the expandable layer.

Like the active agent composition 22, the light push layer 24 may also include a tableting lubricant 34 (represented as a hexagon in FIG. 1). The tableting lubricant 34 within the light push layer 24 can be the same or different as the tableting lubricant 34 used in the active agent composition 22. Typical lubricants suitable for inclusion in the light push layer 24 include, for example, polyethylene glycol, sodium stearate, potassium stearate, magnesium stearate, stearic acid, calcium stearate, sodium oleate, calcium palmitate, sodium laurate, sodium ricinoleate, potassium linoleate, glyceryl monstearate, glyceryl palmitostearate, halogenated castor oil, sodium lauryl sulfate, sodium stearyl fumarate, and zinc stearate. The precise amount of tableting lubricant 34 included in the light push layer 24 will depend upon the lubricant or lubricants used. However, where the tableting lubricants noted herein are used in the light push layer 24, it is presently preferred that the light push layer 24 include between about 0.01 mg and 10 mg of lubricant 34.

Both the light push layer 24 and the active agent composition 22 may include a nontoxic colorant or dye 42 (represented in FIG. 1 by a circle). The colorant 42 may provide the dosage form 10 of the present invention with a more aesthetically pleasing appearance. Moreover, a colorant 42 may serve to identify the dosage form 10 during manufacture or in anticipation of administration. Colorants suitable for use in the light push layer 24 or active agent composition 22 include, for example, ferric oxide red, ferric oxide yellow, ferric oxide green, ferric oxide black, FD&C (Food, Drug, and Cosmetic Act) dyes such as Blue #1 (brilliant blue FCF), Green #6 (quinizarine green SS), Red #22 (eosine), and yellow #8 (uranine), pharmaceutical dyes diluted with aluminum oxide, and the like. The amount of colorant formulated within the light push layer 24 or the active agent composition 22 will depend upon the desired color intensity. Typical levels of use are 0.5 wt% to 2 wt% colorant based on the weight of the material layer into which the colorant is incorporated.

To inhibit oxidation, both the light push layer 24 and the active agent composition 22 either layer may be formulated to include an antioxidant 44 (represented by right slanted dashes in FIG. 1). The antioxidant 44 slows, or prevents the oxidation of the dosage form 10 and its ingredients by atmospheric oxygen. Representative antioxidants that may be included in the light push layer 24 or the active agent composition 22 include, for example, absorbic acid, fumaric acid, asorbyl palmitate, butylated hydroxyanisole, a mixture of 2 and 3 tertiary-butyl-4-hydroxyanisole, butylated hydroxytoluene, sodium isoascorbate, dihydroguaretic acid, sodium ascorbate, sodium metabisulfite, potassium ascobate, vitamin E, propyl gallate, malic acid, 4-chloro-2-,6-ditertiary butylphenol, alphatocopheral, and propylgalate. Where both the active agent composition 22 and the light push layer 24 include and antioxidant 44, the antioxidant used may be the same in both material layers or different, and more than one different antioxidant 44 may be used in each material layer. If used, the antioxidant 44 preferably accounts for up to about 5 mg of the light push layer 24 or up to about 10 mg of the active agent composition 22.

As is easily appreciated, both the active agent composition 22 and the light push layer 24 contribute to the osmotic activity of the osmotic core 20 included in the dosage form 10 of the present invention. Significantly, both the active agent composition 22 and the light push layer 24 are formulated to provide an osmotic core having a desired initial osmotic pressure. As will be explained further, the initial osmotic pressure generated by the osmotic core 20 is important to the proper function of the dosage form 10 of the present invention. The precise magnitude of the initial osmotic pressure generated by the osmotic core 20, however, will vary from application to application, and depends on, among other factors, the amount and type of active agent 28 to be delivered, the desired active agent delivery profile, and the physical or chemical characteristics of the semipermeable membrane 14 and the osmosenstive membrane 16 included in the bi-layer membrane system 12. Nevertheless, in each embodiment of the dosage form 10 of the present invention, the active agent composition 22 and light push layer 24 are formulated to generate an initial osmotic pressure that facilitates efficient, controlled delivery of the active agent 28 included in the active agent composition 22, given the particular semipermeable membrane 14 and osmosensitive membrane 16 used in the bi-layer membrane system 12.

As is illustrated in FIG. 1 and FIG. 2, the active agent composition 22 and light push layer 24 included in the osmotic core 20 of the dosage form 10 of the present invention are manufactured as discrete layers, which may be accomplished using standard manufacturing techniques. For example, the ingredients forming the active agent composition 22 may be blended or blended and pressed into a solid or semi-solid layer. The ingredients forming the active agent composition 22 may be blended with a solvent and formed into a solid or semi-solid formed by conventional methods, such a ball-milling, calendaring, stirring, or roll-milling, and then pressed into a selected shape. The active agent composition 22 will generally be formed into a desired shape having dimensions that correspond to the internal dimensions of the volume the active agent composition 22 is to occupy within the internal compartment 18 of the dosage form 10 of the present invention. The formed active agent composition 22 may then be placed in contact with the light push layer 24, which may be prepared and formed using a process similar to the process used to blend and form the active agent composition 22. Like the active agent composition 22, the light push layer 24 will generally be formed into a shape having dimensions corresponding to the internal dimensions of the volume of the internal compartment 18 to be occupied by the expandable layer. The layering of the active agent composition 22 and the light push layer 24 can be achieved by conventional press-layering techniques.

In another manufacture, the osmotic core is manufactured by a wet granulation technique. In a wet granulation technique the active agent and the ingredients comprising the active agent composition are blended using a solvent, such as isopropyl alcohol as the granulation fluid. Other granulating fluid, such as water or denatured alcohol, can be used for this purpose. The ingredients forming the active agent composition are individually passed through a 40 mesh screen and then thoroughly blended in a mixer. Next, other ingredients comprising the active agent composition are dissolved in a portion of the granulation fluid, such as the solvent described above. Then, the latter prepared wet blend is slowly added to the active agent blend with continual blending in the mixer. The granulating fluid is added until a wet blend mass is produced, which wet mass is then forced through a 20 mesh screen onto oven trays. The extruded blend is dried for 18 to 24 hours at 25°C to 40°C, typically in forced air, to remove granulating solvent. The dry granules are then screened with a 16 mesh screen. Next, a lubricant is passed through a 60 mesh screen and added to the dry screened granule blend. The granulation is then put into mixing containers and tumble-mixed for 1 to 10 minutes. This general procedure may also be followed to prepare a granulation for the light push layer. After granulations for both the active agent composition and the light push layer are prepared, the active agent composition and light push layer may be layered and pressed into a layered tablet using a suitable layer press, such as, for example, a Korsch or Manesty® layer press.

Another manufacturing process that can be used for providing the active agent and light push layers includes blending their powdered ingredients in a fluid bed granulator. After the powdered ingredients of the active agent composition and light push layer are dry blended in the granulator, a granulating fluid, for example, poly(vinylpyrrolidone) dissolved or dispersed in a solvent, such as water, is sprayed onto the respective powders. The coated powders are then dried in a granulator. This process coats the ingredients present therein while spraying the granulating fluid. After the granules are dried, a lubricant, such as stearic acid or magnesium stearate, is blended as above into the mixture. The granules may then pressed in the manner described above to form the layered osmotic core.

Generally, the bi-layer membrane system 12 of the dosage form 10 of the present invention is formed around the osmotic core 10 of the dosage form 10 after the osmotic core has been manufactured, resulting in a dosage form 10 including an internal compartment 18 occupied by the osmotic core 20. The physical and chemical characteristics of the semipermeable membrane 14 and the osmosensitive membrane 16 included in the bi-layer membrane system 12 facilitate controlled delivery of the active agent 28 from the active agent composition 22 included in the osmotic core 20. Moreover, the bi-layer membrane system 12 of the dosage form 10 of the present invention provides structural integrity to the dosage form 10 during operation. Therefore, the bi-layer membrane system 12 of the dosage form 10 of the present invention not only contributes to the delivery dynamics of the active agent 28, but the bi-layer membrane system 12 makes controlled delivery of the active agent 28 possible by substantially maintaining the structure of the dosage form 10 as the dosage form 10 functions in the environment of operation.

The semipermeable membrane 14 of the dosage form 10 of the present invention is constructed of a semipermeable composition that is non-toxic to the intended subject and does not adversely affect the performance of the osmosensitive membrane 16, the active agent composition 22, or the light push layer 24. The semipermeable membrane 14 is formed of a material that is permeable to the passage of water or other aqueous fluids but is substantially impermeable to the passage of the active agent 28 included in the active agent composition 22. In addition, the material forming the semipermeable membrane 14 is formulated to maintain its physical and chemical integrity in the intended environment of operation at least for the anticipated operational life of the dosage form 10. That is, the semipermeable membrane 14 does not lose its structure or undergo a chemical change as the active agent 28 is dispensed from the dosage form 10 of the present invention, even as the osmotic core 20 degrades during active agent 28 delivery. During the functional life of the dosage form 10, the permeability of the material forming the semipermeable membrane 14 may vary, but where the material forming the semipermeable membrane 14 exhibits a varying permeability, the magnitude of the change in the permeability of the semipermeable membrane 14 is much smaller than the magnitude of the change in permeability exhibited by the osmosensitive 16 membrane. The semipermeable membrane 14 of the bi-layer membrane system 12, therefore, not only provides structural integrity, but it also facilitates the controlled delivery of the active agent 28 by preventing the escape of active agent 28 from the dosage form 10, except through the delivery passageway 26 provided.

Materials suitable for forming the semipermeable membrane 14 include, for example, a cellulose ester polymer, a cellulose ether polymer, or a cellulose ester-ether polymer. These cellulosic polymers preferably have a degree of substitution ("DS") on the anhydroglucose unit from greater than 0 up to 3, inclusive. Exemplary polymers that may be used to fabricated the semipermeable membrane 14 include, for example, cellulose acylate, cellulose diacylate, cellulose triacylate, cellulose acetate, cellulose diacetate, cellulose triacetate, cellulose triacylate, and mono-, di-, and tricellulose alkinylates. Such polymers include, for example, cellulose acetate having a DS of up to 1 and an acetyl content of up to 31 wt%, cellulose acetate having a DS of 1 to 2 and any acetyl content of 21 to 35 wt%, and cellulose acetate having a DS of 2 to 3 and an acetyl content of 35 to 44 wt%. Additional polymers that may be used in forming the semipermeable membrane include those polymers set forth in U.S. patent 6,245,357. Though the construction of the semipermeable membrane 14 is not limited to the use of such polymers, the polymers listed herein allow fabrication of semipermeable membranes exhibiting the permeability and structural and chemical integrity characteristics necessary to the performance of the dosage form 10 of the present invention.

As is easily appreciated by reference to FIG. 1 and FIG. 2, the osmosensitive membrane 16 included in the bi-layer membrane system 12 is positioned between the osmotic core 20 of the dosage form 10 and the semipermeable membrane 14. The volumetric flow of water, dV/dt, osmotically imbibed by dosage form 10 is expressed by series resistance equation. Each layer imparts resistance to the transport of water as water flows from the environment of use across the bi-layer membrane into the osmotic core.

We find that the permeability of osmoresponsive membrane 16 and semimpermeable membrane 14 are functions of osmotic pressure and that these permeabilities can be measured as a function of osmotic pressure. The measurements are conducted in a Franz cells with reference solutions spanning a range of known osmotic pressures according to the procedures detailed in US 6,245,357. Permeability measurements represent the average values collected over a period of time, typically between 4 hours and 9 hours. The average permeability, k, of each layer can then be expressed mathematically in terms of osmotic pressure.

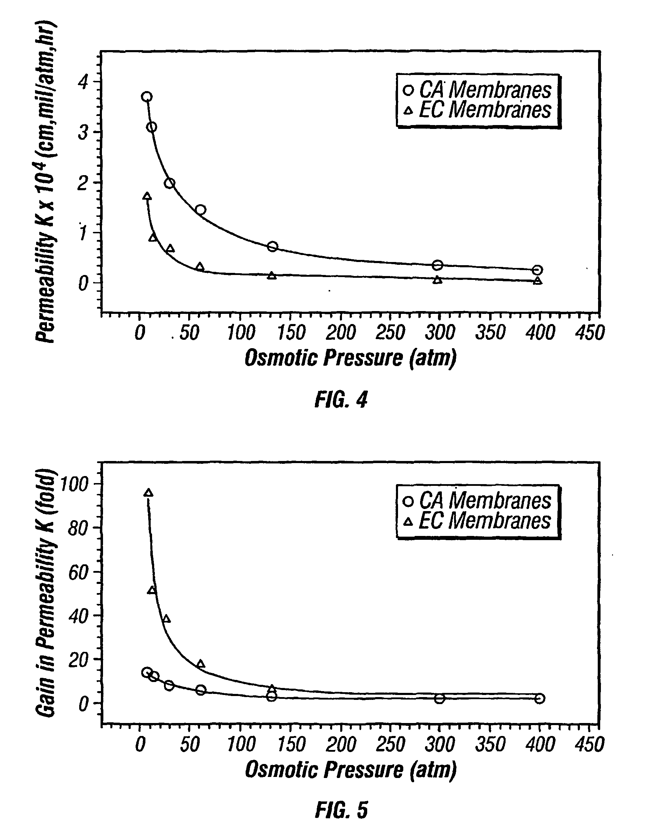

The triangular symbols in FIG.4 represents a plot of the permeability $k_1$ of a representative osmosensitive membrane 16 as a function of osmotic pressure. The circular symbols in FIG.4 represent the permeability $k_2$ of a representative semipermeable membrane 14 as a function of osmotic pressure. Both the osmosensitve membrane 16 and the semipermeable membrane 14 become more permeable as the osmotic pressure declines. The semipermeable membrane 14 is more permeable than the osmosensitive membrane 16 at any given osmotic pressure.

FIG. 5 illustrates these permeability data for the same semipenneable membrane 14 and the same osmosensitive membrane 16 used to generate the date plotted in FIG. 4, except in FIG. 5, the permeability date is plotted in terms of the gain in permeability of each membrane as a function of osmotic pressure. The gain is referenced to the permeability of each membrane when immersed in a saturated solution of sodium chloride solution having an osmotic pressure of about 400 atmospheres. This plot reveals, surprisingly, that the gain in permeability of the osmosensitve membrane 16 increases much more as osmotic pressure declines compared to the gain in permeability of semipermeable membrane 14 under the same osmotic conditions. Therefore, the permeability of the osmosensitive membrane 16 substantially increases in response to a decrease in the osmotic pressure exerted by the material present in the internal compartment 18 (the "internal osmotic pressure"). Referring to Equation 1, it is apparent that as the permeability of the osmosensitvie layer 16 increases, the rate of water imbibed across the bi-layer membrane system 12 increases. Therefore, while the dosage form 10 is in operation and while the internal osmotic pressure of the dosage form 10 declines, the permeability of the bi-layer membrane system 12 increases at an accelerating rate, providing a corresponding acceleration in the hydration rate of the light push layer 24 and the active agent composition 22 as the active agent 28 is delivered from the dosage form 10.

Advantageously, the permeability of each layer can be increased or decreased by increasing or decreasing the amount of flux enhancer blended into each layer. For example, the value of $k_2$ in semipermeable membrane layer 16 composition of cellulose acetate can be increased by increasing the amount of triblock copolymer blended into the semipermeable membrane composition or decreased by decreasing the amount of triblock copolymer blended into the semipermeable membrane composition.

To achieve an osmosensitive membrane having a permeability that responds to changes in the magnitude of the osmotic pressure gradient generated across the bi-layer membrane system 12, the osmosensitive membrane 16 is formulated using a hydrophobic substance 46 (identified by filled triangles in FIG. 1) and a hydrophilic substance 48 (identified by wavy lines in FIG. 1). Though the hydrophobic substance 46 of the osmosentive membrane is substantially impermeable to the passage of aqueous fluid, the hydrophilic substance swells within the osmosensitive membrane in the presence of aqueous fluid. Swelling of the hydrophilic substance 48 creates avenues for fluid passage, or pores, and causes the permeability of the osmosensitive membrane 16 to increase as osmotic pressure decreases. The hydrophilic substance 48 included in the osmosensitive membrane 16, is chosen such that the extent of swelling of the hydrophilic substance 48 is osmosensitive or osmoresponsive. The terms "osmoreponsive" and "osmosensitive" are used interchangeably to describe the swelling behavior of hydrophilic substance 48 in response to osmotic pressure. Specifically, swelling of the hydrophilic substance 48 increases in response to a decrease in internal osmotic pressure of the dosage form 10 of the present invention.

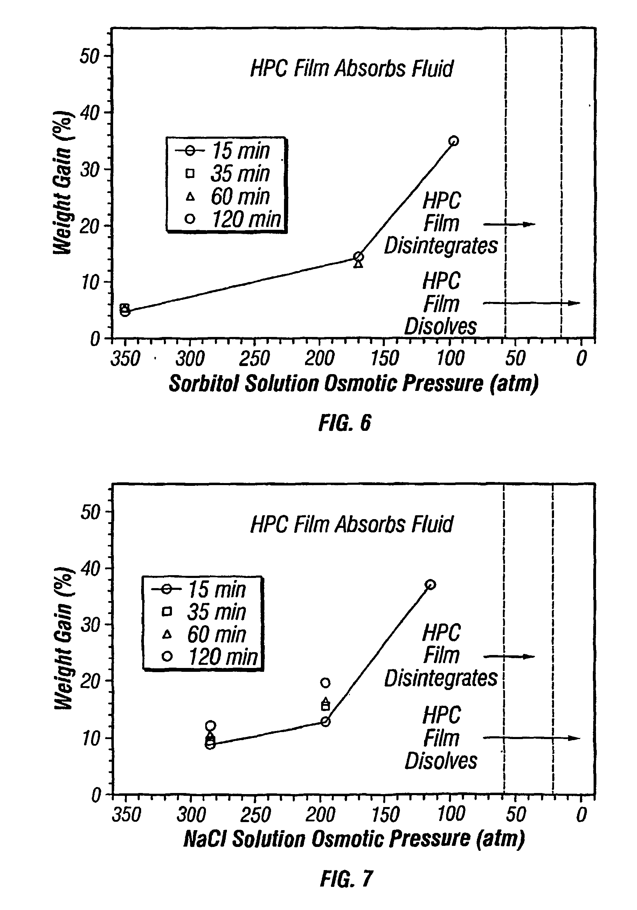

FIG. 6 illustrates the swelling behavior of hydroxypropyl cellulose, an osmosensitive material that may be used as the hydrophilic substance 48 included in the osmosensitive membrane 16 of the bi-layer membrane system 12. Swelling is measured as weight gain of a film of hydroxypropyl cellulose after being immersed for a known duration of time in a series of solutions spanning various experimentally pre-measured osmotic pressures. The solutions were prepared by dissolving an osmoagent, sorbitol in this instance, in a series of concentrations in de-ionized water. The solutions were maintained at a constant temperature of 37° C during the experimental testing. Swelling of the hydroxypropyl cellulose increased as the osmotic pressure of the solutions decreased, and as the osmotic pressure declined to approximately 60 atmospheres and below, the films became so swollen and so softened that they disintegrated and dissolved. The weight of the films at a fixed osmotic pressure of 170 atmospheres and of 350 atmospheres was also monitored as a function of time. As can be appreciated by reference to FIG. 6, once the exemplary hydroxypropyl cellulose film was immersed in a sorbitol solution of a given osmotic pressure, the weight of the film did not change substantially between 15 minutes and 120 minutes after immersion.

FIG. 7 illustrates the swelling behavior of hydroxypropyl cellulose films while immersed in solutions of a different osmoagent, sodium chloride. As it does in solutions of sorbitol, the hydroxypropyl cellulose film swells to a greater extent in sodium chloride solutions exhibiting relatively lower osmotic pressures. Surprisingly, however, we also find that the swelling of the hydroxypropyl cellulose film has a time dependency in solutions of sodium chloride, a behavior not exhibited when the hydroxypropyl cellulose film is immersed in aqueous sorbitol solutions. Specifically, hydroxypropyl cellulose films immersed in aqueous sodium chloride solutions of fixed osmotic pressure continue to substantially swell with time. The swelling of osmosensitive hydroxypropyl cellulose film immersed in a sodium chloride solution having fixed osmotic pressure of 200 atmospheres, for example, continuously increases between 15 minutes, 35 minutes, 60 minutes, and 120 minutes (shown in FIG. 7, with increased swelling as a function of time reflected in the circular, square, triangular, and diamond symbols provided in the graph). Therefore, the extent of swelling of the hydrophilic substance 48 included in the osmosensitive membrane 16 of the dosage form 10 of the present invention may increase as a function of both time and osmotic pressure where sodium chloride is included in the osmotic core 20 of the present invention and is in direct contact with the osmosensitive membrane 16.

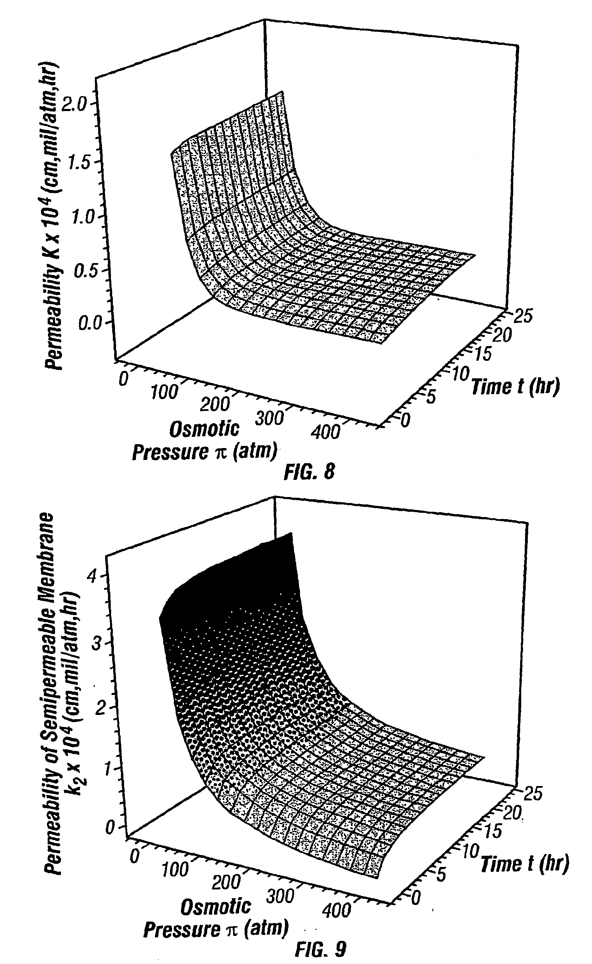

Where the bilayer membrane system 12 includes an osmosensitive membrane 16 formulated using hydroxypropyl cellulose as a hydrophilic substance 48, the combined effect on the permeability of the osmosensitive membrane 16 as a function of osmotic pressure and time when exposed to a sodium chloride solution is represented in the three-dimensional plot provided in FIG. 8. Likewise, the combined effect of osmotic pressure and time in sodium chloride solution on the permeability of semipermeable membrane 14 is represented in the three-dimensional plot FIG. 9.

Regardless of the type of osmotic agent present in the osmotic core 20 of the dosage form 10 of the present invention, hydrophilic substance 48 included in the osmosensitive membrane 16 swells to a greater extent at lower osmotic pressures, causing the permeability of the osmosensitive membrane 16 of the bi-layer membrane system 12 to increase in response to a decrease in the internal osmotic pressure over the functional life of the dosage form 10. Moreover, use of sodium chloride as an osmoagent provides an unexpected and independent parameter useful in the present invention. When sodium chloride is formulated within the osmotic core 20 of the dosage form 10, ther permeability of the osmosensitive membrane 16 and, therefore, the bi-layer membrane system 12 may increase both as a function of time (even while the internal osmotic pressure of the dosage form is substantially fixed) and as a function of internal osmotic pressure.

In a preferred embodiment, the osmosensitive membrane 16 of the bi-layer membrane system 12 includes a blend of ethycellulose and a hydroxyalkylcellulose comprising an alkyl group of 1 to 5 carbons, such as hydroxypropylcellulose. An osmosensitive membrane made of a blend of ethylcellulose and a hydroxypropylcellulose will generally include a blend of about 40 wt% to about 99 wt% ethylcellulose and about 1 wt% to about 60 wt% of the hydroxyalkylcellulose, with the total weight of the blend equal to 100 wt%. The ethylcellulose used in the preferred osmosensitive membrane includes 15 wt% to 60 wt% ethoxy content, is characterized by a viscosity of about 4 centipoises to about 200 centipoises, or higher, exhibits a number-average molecular weight of about 5,000 to about 1,250,000, and is nontoxic, substantially insoluble in water, and substantially insoluble in gastrointestinal fluid. The hydroxyalkylcellulose in the preferred osmosensitive membrane preferably possesses a number-average molecular weight of about 7,500 to 1,500,000, is also nontoxic, and is soluble in water below 40°C and in ethyl alcohol. Additionally, other components can be incorporated within the osmosensitve membrane 16 composition such as for example a surfactant. The surfactant serves to compatibilize the hydrophilic hydroxyalkylcellulose to the hydrophobic alkylcellulose. Such compatibilizers typically comprise a hydrophilic moiety and a hydrophobic moiety within the molecular structure of the surfactant. Preferred compatibilizers are those that dissolve in the same solvent as is used to dissolve the ethyl cellulose and the hydroxyalkylcellulose to form the coating solution in the membrane coating processes. For example, a surfactant of polyoxyl 40 stearate that provides the hydrophilic moiety in the polyoxyl fraction and the hydrophobic moiety in the stearate group, can be used as a compatibilizer in the osmosensitive membrane 16. Alternately, polyoxyl 50 stearate can be used as the compatibilizer. Yet another class of surfactant compatibilizers useful in forming the osmosenstive membrane is triblock co-polymers of ethylene oxide/propylene oxide/ethylene oxide, also known as poloxamers. In this class of surfactants, the hydrophilic ethylene oxide ends of the surfactant molecule and the hydrophobic midblock of propylene oxide of the surfactant molecule serve to compatibilize the ethylcellulose and hydroyalkyl cellulose. Other compatibilizing surfactants include polyoxyethylene 23 lauryl ether, polyoxyethylene 23 lauryl ether, polyoxyethylene 20 cetyl ether, polyoxyethylene 20 stearyl ether, polyoxyethylene 100 stearyl ether, polyoxyethylene 10 oleyl ether, polyoxyethylene 100 stearate, polyoxyethylene 20 sorbitan monolaurate, polyoxyethylene 20 sorbitan monopalmitate, polyoxyethylene 20 sorbitan monostearate, polyoxyethylene 20 sorbitan monooleate, and the like. Specific membrane compositions utilizing these compatibilizers include membranes sprayed from a solvent system including ethyl alcohol or ethyl alcohol Formula SDA3A.

An exemplary composition for providing an osmosensitive membrane 16 useful in the bi-layer membrane system 12 of the dosage form 10 of the present invention includes a composition of ethyl cellulose, hydroxypropyl cellulose, and a surfactant or a blend of surfactants. In a preferred embodiment, the osmosensitive membrane 16 of the bi-layer membrane system 12 includes 1 wt% to 30 wt% surfactant, with the 70 wt% to 99 w% balance of the osmosensitive membrane 16 consisting of the blend of ethyl cellulose and hydroxypropyl cellulose. In another embodiment, the osmosensitive membrane 16 includes 40 wt% to 80 wt% percent ethyl cellulose, 10 wt% to 50 wt% hydroxypropyl cellulose, and 1 wt% to 30 wt% compatibilizing surfactant.

The osmosensitive membrane 16 of the dosage form 10 of the present invention, however, is not limited to membranes constructed using ethylcellulose and a hydroxyalkylcellulose. The hydrophobic substance 46 included in the osmosensitive membrane 16 may include, for example, alkyl alcohols such as cetyl alcohol or stearyl alcohol, polyurethanes, silicones, polystyrene, phenol-formaldehyde resins, polyamides, ethylene vinyl acetate, polyvinyl acetate, ethylene vinyl acetate copolymers, polymethylmethacrylate, ethyl acrylate methyl methacrylate copolymer, cellulose butyrate, cellulose nitrate, cellulose acetate with an acetyl content of greater than 20 wt%, cellulose acetate phthalate, cellulose acetate propionate, cellulose triacetate, ethyl hydroxy ethyl cellulose, ethyl acrylate methyl methacrylate copolymer, poly(butyl methacrylate (2-dimethyl aminoethyl)methacrylate, methyl methacrylate), methacrylic acid methylmethacrylate copolymer, chitan, chitosan, rosin ester gums, shellac, zein, and the like. Additionally, the hydrophilic substance 48 may include, for example low substituted hydroxypropyl cellulose, hydroxypropyl methylcellulose, methylcellulose, hydroxyethyl methylcellulose, polyvinyl pyrrolidone, polyvinyl acetate polyvinyl pyrrolidone copolymer, gelatin, starch, polyethylene glycol polyvinyl alcohol copolymer, carrageenan, algin, agar, gum acacia, gum karyara, carob bean gum, gum tragacanth, gum ghatti guar gum, caseinates, cellulose acetate with an acetyl content of less than 20 wt%, sodium carboxymethyl cellulose, potassium carboxy methyl cellulose, polyvinyl alcohol, polyvinyl alcohol polyethylene glycol graph copolymers, cellulose acetate phthalate, hydroxypropyl methycellulose phthalate, hydroxypropyl methyl cellulose acetate succinate, or any blends, molecular weights, or combinations of each, as desired. It should also be emphasized that the osmosensitive membrane 16 may be formulated using more than one different hydrophobic substance 46 or more than one different hydrophilic substance 48.

The osmosensitive 16 and semipermeable membranes 14 of the bi-layer membrane system 12 of the dosage form 10 of the present invention may be manufactured using known coating techniques. For example, the membranes of the bi-layer membrane system 12 may be formed around the osmotic core 20 using any suitable molding, spraying, or dipping techniques. Alternatively, the membranes of the bi-layer membrane system 12 may be formed around the osmotic core 20 using a known air-suspension process. Air suspension processes are well suited to independently forming each membrane of the bi-layer membrane system 12 and generally consist of suspending and tumbling a membrane-forming composition in a current of air until a membrane of desired thickness is applied to the osmotic core 20. Where an air suspension procedure is used to form the bi-layer membrane system 12, the osmosensitive membrane 16 may be formed using, for example, ethanol as a solvent, and the semipermeable membrane 14 may be formed using, for example, an organic solvent, such as acetone-water cosolvent 90:10 to 100:0 (wt:wt) with 2.5 wt% to 7 wt% polymer solvents. Commercially available air suspension coaters, such as a Wurster® air suspension coater, an Aeromatic® air suspension coater, or a Glatt® air suspension coater, can be used for applying both membranes of the bi-layer membrane system 12.

Yet another technology that may be used to fabricate the bi-layer membrane system 12 is pan coating. In a pan coating system, membrane-forming compositions are deposited by successive spraying of the compositions used to form the osmosensitive membrane 16 and the semipermeable membrane 14, with the spraying of the compositions being accompanied by tumbling in a rotating pan. A larger volume of cosolvent can be used in a pan-coating procedure, and an increase in cosolvent volume results in a reduced concentration of polymer solids and facilitates the production of thinner and more uniform membrane structures. Once the osmotic core 20 is coated with the bi-layer membrane forming compositions, the bi-layer membrane system 12 is generally mechanically or laser drilled to create the delivery passageway 26 and then dried in a forced air or humidity oven for 1 to 3 days, or longer, to free the solvent used in coating process. Generally, membranes formed by air suspension or pan-coating technologies have a thickness of 2 to 20 mils (0.051 to 0.510 mm) with a presently preferred thickness of 2 to 10 mils (0.051 to 0.254 mm).

The delivery passageway 26 included in the dosage form 10 of the present invention may include any suitable aperture, orifice, bore, pore, or porous element through which the active agent 28 included in the active agent composition 22 can pass. Though the delivery passageway 26 is preferably formed as an orifice through the bi-layer membrane system 12 using a mechanical or laser drilling process, the delivery passageway 26 may also include a fiber, capillary tube, porous overlay, porous insert, microporous member, or porous composition. Further, the delivery passageway 26 can have any shape, such as round, triangular, square or elliptical, for assisting in the controlled release of the active agent 28. The dosage form 10 of the present invention can also be manufactured with more than one delivery passageway, such as, for example, two, three, four, or more passageways, in spaced apart relation through the bi-layer membrane system 12. If desired, the delivery passageway 26 included in the dosage form 10 of the present invention may also be formed using a material that erodes or is leached away in the presence of aqueous fluid to produce at least one delivery passageway 26. Representative leachable or credible materials that may be used in forming a delivery passageway for the dosage form 10 of the present invention include, for example, poly(glycolic) acid, poly(lactic) acid, a gelatinous filament, a water-removable poly(vinyl alcohol), sorbitol, sucrose, lactose, maltose or fructose, or other leachable compounds, such as fluid-removable, pore-forming polysaccharides, acids, salts, or oxides. Representative materials or compounds for forming a delivery passageway as included in the dosage form 10 of the present invention are disclosed in the following references: U.S. Patent No. 3,845,770 and U.S. Patent No. 3,916,899, both to Theeuwes and Higuchi; U.S. Patent No. 4,063,064 to Saunders et al.; and U.S. Patent No. 4,088,864 by Theeuwes et al. Delivery passageways formed by aqueous leaching are disclosed in U.S. Patent No. 4,200,098 and U.S. Patent No. 4,285,987, both to Ayer and Theeuwes.

Although it is not illustrated in the figures, the dosage form 10 of the present invention may also include an overcoat on the outer surface of the bi-layer membrane system 12. The overcoat may be a therapeutic composition including, for example, 0.5 to 200 mg of a second active agent and 0.5 to 400 mg of a pharmaceutically acceptable carrier. The carrier included in such and overcoat may include, for example, hydroxypropyl methylcellulose, hydroxyethyl cellulose, polyvinyl pyrrolidone, vinyl acetate polypyrrolidone copolymer, polyethylene glycol polyvinylpyrrolidone copolymer, polyethylene glycol polyvinyl alcohol graft copolymer, and the like. The overcoat, can be formulated with 0 to 50 wt % of a plasticizer, opacifier, colorant, or anti-tack agent. Where included, the overcoat preferably provides therapy immediately as the overcoat dissolves or undergoes dissolution in the presence of an aqueous fluid, such as gastrointestinal fluid, and concurrently therewith delivers the second active agent included in the overcoat. The second active agent formulated into the overcoat may be different from or the same as the active agent 28 included in the active agent composition 22 included in the osmotic core 20 of the dosage form 10. Moreover, where an overcoat is provided, the overcoat may include more than one active agent, for example, the overcoat may incorporate second and third active agents, or second, third, and fourth active agents, as desired.

FIG. 3 illustrates the dosage form 10 of the present invention in operation during an active agent delivery period. When initially placed into an aqueous environment, or into a fluid biological environment, such as the gastrointestinal tract of a chosen subject, the dosage form 10 of the present invention begins to imbibe aqueous fluid due to the osmotic pressure exerted by the osmotic core 20. If the dosage form 10 of the present invention includes an outer coating, however, that outer coating may need to dissolve, at least partially, before aqueous fluid begins to enter the dosage form 10 through the bi-layer membrane system 12. As aqueous fluid is taken into the dosage form 10, both the active agent composition 22 and the light push layer 24 hydrate. Hydration of the active agent composition 22 converts the active agent composition 22 into a solution, a liquid, gel, or gel-like substance that is deliverable through the delivery passageway 26, while hydration of the light push layer 24 causes the osmopolymer 38 included in the light push layer 24 to expand and expel the hydrated active agent composition 22. Osmotic and hydrostatic forces also develop within the dosage form 10 of the present invention and, because the active agent 28 cannot pass through the semipermeable membrane 14 of the bi-layer membrane system 12, these forces also contribute to the expulsion of active agent 28 through the delivery passageway.

Despite the relatively large osmotic pressure initially exerted by the osmotic core 20 of the dosage form 10 of the present invention, hydration rate of the active agent composition 22 and light push layer 24 included in the osmotic core 20 starts relatively slowly. This is because the relatively large osmotic pressure initially exerted by the osmotic core 20 results in a relatively low permeability across the osmosensitive membrane 16. The low initial rate of hydration of the active agent composition 22 and light push layer 24 results in an initially slower hydration rate of these layers corresponding to a slower release rate of the active agent 28.

As active agent 28 is delivered from the dosage form 10 and the osmotic core materials become increasingly hydrated, however, the osmotic pressure exerted by the osmotic core 20 decreases as the components of the osmotic core become diluted by water entering osmotic core 20. The decreasing osmotic pressure exerted across the bi-layer membrane system 12 results in an increase in the swelling of the hydrophilic substance 48 included in the osmosensitive membrane 16, which results in a net increase in the permeability of the bi-layer membrane system 12. Thus, as active agent 28 is delivered from the dosage form 10 of the present invention, the rate at which the active agent composition 22 and light push layer 24 hydrate accelerates, allowing the dosage form to achieve a relatively higher and desireable rate of release of the active agent 28 for prolonged time. Although the internal osmotic pressure of the dosage form 10 will decrease during delivery of the active agent 28, the dosage form 10 of the present invention is still capable of providing a zero order or ascending release of the active agent 28 over an extended period of time. The accelerating rate of hydration of the active agent composition 22 and light push layer 24 that occurs as the osmotic activity of the osmotic core materials decreases effectively offsets any decrease in delivery rate that would otherwise occur if the dosage form 10 was provided only a membrane of substantially fixed permeability.

Significantly, it has been found that the permeability of the osmosensitive membrane 16 of the bi-layer membrane system 12 is related exponentially, as described by Equation 2, to the magnitude of the osmotic pressure gradient exerted across the osmosensitive membrane 16, and the osmosensitive membrane 16 also exhibits a threshold permeability behavior. That is, the permeability of the osmosensitive membrane 16 increases very slowly until a certain osmotic pressure threshold, or permeability threshold, is reached. Once the osmotic pressure gradient decreases to or below the permeability threshold, the permeability of the osmosensitive membrane 16 increases steeply. The permeability threshold for a given osmosensitive membrane included in the dosage form 10 of the present invention may be exhibited either at a particular osmotic pressure or it may be exhibited over a range of osmotic pressures. The permeability threshold for the osmosenstive membrane 16 formulated with hydrophilic substance 48 hydroxypropyl cellulose of molecular weight 80,000 grams per mole, for example, is triggered at an osmotic pressure value of approximately 100-150 atmospheres. Moreover, the permeability threshold for the osmosensitive membrane 16 included in the bi-layer membrane system 12 of the dosage form 10 of the present invention will vary depending on the specific formulation used to fabricate the osmosensitive membrane 16. For example, the permeability threshold may vary as either the amount or type of the hydrophobic material 46 or the hydrophilic material 48 used in the osmosensitive membrane 16 are varied.

An appreciation of the threshold permeability behavior of the osmosensitive membrane 16 of the dosage form 10 of the present invention is significant because it enables the fabrication of the dosage form 10 of the present invention using only a light push layer 24. The dosage form 10 of the present invention is capable of providing controlled release of even insoluble active agents using only a light push layer 24 because the dosage form 10 is formulated such that, as the active agent 28 is delivered from the dosage form 10, both the light push layer 24 and the active agent composition 22 are hydrating at accelerating rates during the functional life of the dosage form. The accelerating rates of hydration facilitate complete delivery of the active agent 28 using only a light push layer 24 because the deliverability of the active agent 28 within the active agent composition 22 and the rate of expansion of the light push layer 24 both increase in response to increases in the rate of hydration. In order to achieve a dosage form that provides accelerating rates of hydration of the osmotic core materials as the active agent 28 is delivered, however, the permeability of the bi-layer membrane system 12 must increase while the osmotic activity of osmotic core materials included in the dosage form 10 is sufficiently high to cause an increase in fluid flux in response to the increase in permeability. It has been found that these conditions are met if the dosage form 10 of the present invention is formulated such that the initial osmotic pressure exerted by the osmotic core 20 is at or exceeds the permeability threshold of the osmosensitive membrane 16 included in the dosage form 10. If the initial osmotic pressure exerted by the osmotic core 20 of the dosage form 10 does not fall within or exceed the permeability threshold of the osmosensitive membrane 16, the osmotic driving pressure exerted by the materials froming the osmotic core 20 will not be sufficient to provide complete delivery of the desired dose of active agent 28 at controlled rates over a prolonged period of time. Where the osmotic core 20 is formulated to exert an initial osmotic pressure greater than the permeability threshold of the osmosensitive membrane 16, however, the extent to which the initial osmotic pressure exceeds the permeability threshold must be carefully monitored. If the initial osmotic pressure exerted by the osmotic core causes the internal osmotic pressure of the dosage form to remain too high for too long a duration of time, the osmotic pressure gradient across the osmotic core will not decrease quickly enough to allow complete delivery of the active agent 28 during the operational life of the dosage form 10. Therefore, an understanding of the threshold permeability behavior of the osmosensitive membrane 16 is key to formulating and fabricating an osmotic core 20 that ensures efficient, controlled delivery of the active agent 28 using only a light push layer 24.

To take advantage of the threshold permeability behavior of the osmosenstive membrane 16 included in the dosage form 10 of the present invention, then, the active agent composition 22 and the light push layer 24 included in the osmotic core 10 are formulated to exert an initial osmotic pressure that is at or exceeds the permeability threshold for the osmosensitive membrane 16. For example, if the osmosensitive membrane 16 used in the dosage form 10 of the present invention exhibits a permeability threshold at between about 100 atm and 150 atm, the osmotic core materials included in the dosage form 10 will be formulated to exhibit an initial osmotic pressure that is in the 100 atm to 150 atm range, or greater. If a delay in release is desired, the osmotic core materials may be formulated to exert an initial osmotic pressure that is greater than the permeability threshold for the osmosensitive membrane 16 so that a time delay of desired magnitude occurs before significant amounts of active agent 28 are delivered. The magnitude of the delay is easily controlled, with the delay increasing as the initial osmotic pressure increases away from the applicable permeability threshold but decreasing as the initial osmotic pressure approximates the applicable permeability threshold.

As is easily appreciated, the controlled release of active agent 28 achieved by the dosage form 10 of the present invention is dependent upon the physical and chemical characteristics of each of the semipermeable membrane 14, the osmosensitive membrane 16, the active agent composition 22, and the light push layer 24. For example, the amount or type of osmopolymer 38 or hydrogel 30 included in the light push layer 24 or active agent composition 22, respectively, may be varied in order to achieve a different active agent delivery profile, even if the permeability profile of the bi-layer membrane system 12 remains substantially the same. Moreover, the materials used to create both the semipermeable membrane 14 and the osmosensitive membrane 16 are easily varied to provide a bi-layer membrane system 12 that provides a permeability profile suited either to the delivery of a particular active agent or to the delivery of a given active agent at a desired release rate.

The design flexibility of the dosage form of the present invention allows the functional advantages of provided by the dosage form to be applied to the delivery of a wide range of active agents. Therefore, though the dosage form of the present invention is described herein with reference to various figures, materials, and examples, such references are meant only to facilitate an understanding of the invention and, as will be understood by one of skill in the art, do not limit the present invention to any specific embodiment detailed herein.

EXAMPLE 1

An exemplary osmosensitive membrane according to the present invention was fabricated using ethycellulose ("EC") and hydroxypropyl cellulose ("HPC"). The osmosensitive membrane of this example included, by weight percent, 55% ethylcellulose, 40% hydroxypropyl cellulose, and 5% of a compatibilizing surfactant, polyethylene glycol 40 stearate. The ethyl cellulose had an ethoxyl content of 48.0 to 49.5 wt%, a number-average molecular weight of approximately 220,000 grams per mole, a degree of substitution of 2.46 to 2.58, and a viscosity value of 100 centipoise as a measured in a 5 wt% solution dissolved in 80 parts toluene and 20 parts ethanol. The EC material is supplied as Ethocel Standard Premium 100 cps by Dow Chemical of Midland, Michigan. The hydroxypropyl cellulose had molecular weight of approximately 80,000 grams per mole and a viscosity of 300 to 700 centipoise as measured in a 10 wt% solution in water. This osmoresponsive material is supplied as Klucel®EFX and is manufactured by Aqualon of Wilmington, Delaware. The polyethylene glycol 40 stearate is available commercially as MYRJ® 52S and is supplied by Uniqema, of New Castle, Delaware or as PEG-40 stearate as supplied by A. & E. Connock, LTD, Hampshire, England. Myrj 52S consists of mixture of monesters and di-esters of stearic acid with mixed polyethylene diols, the average polymer length being equivalent to about 40 oxyethylene units and an average molecular weight of the surfactant is approximately 2,050 grams per mole.

This EC/HPC osmosensitive membrane was fabricated using a spray-forming process. The process involved first dissolving 17.5 grams of Myrj 52S in 4,650 grams of in specially de-natured alcohol formula SDA3A anhydrous with warming to 35 degrees centigrade and stirring for 15 minutes. 140 grams of HPC was passing through a sieve with 12 wires per inch to de-lump the material. The sieved HPC was then blended with 192.5 grams of EC. The blend of dry powders was added slowly with stirring to the ethanolic surfactant solution and stirred for four hours. The solution was allowed to stand for 3 days to provide homogenous solvation and dissolution of the components. The resulting mixture was then sprayed coated onto plastic discs in a Vector coater fitted with a 12-inch pan. The coating pan was charged with tablets as fillers to provide bulk for good tumbling action so the discs could be coated uniformly. The plastic discs had a diameter of about 1 inch and were fabricated of Delrin. In this pan coating process, the solids of the coating solution are sprayed onto the discs while the solvent was simultaneously removed and exhausted in a current of warm air. This coating process continued until a coating thickness of 4-7 mils osmosensitive membrane was accumulated onto the surfaces of the discs.

Next, a semipermeable membrane was spray applied onto osmosensitive membrane in the pan coating operation. A coating solution was first prepared by dissolving 50 grams of poloxamer and 200 grams of cellulose acetate in 4,750 grams of acetone with warming and stirring. The cellulose acetate had an average acetyl content of 39.8 wt% and a number average molecular weight of approximately 40,000 grams per mole, and a falling ball viscosity of 10 seconds. The cellulose acetate is commercially available as CA-398-10 from Eastman Chemical Company of Kingsport, Tennessee. Poloxamers are a:b:a tri-block co-polymers with monomer repeat units consisting of ethylene oxide: propylene oxide: ethylene oxide. The grade formulated in this semipermeable membrane had monomer ratios of 80:27:80. This poloxamer has an average molecular weight ranging from 7,680 to 9,510 grams per mole and is commercially available as Lutrol F68 ("Lutrol") from BASF Corporation of Parsippany, New Jersey. The resulting coating solution was sprayed onto the bed of tablets and discs previously coated with osmosenstive membrane until a thickness of 1 to 3 mils was accumulated. Finally, resulting bi-layer membrane system was peeled from the discs and dried to remove residual coating solvent.

In a separate coating operation, a fresh bed of tablets and discs were coated with the solution of cellulose acetate and poloxamer until 4 to 7 mils of semipermeable membrane was accumulated onto the discs. The resulting single layer semipermeable membrane was then peeled from the discs and dried.

The resulting dried single layer semipermeable membranes were mounted in Franz cells. Distilled water was placed on one side of the membrane and sodium chloride solution of known concentration and osmotic pressure was present on the opposite side. The osmotic pressure of the solution had been previously measured in a Knauer vapor pressure osmometer. A graduated pipet was fitted to the cell containing the sodium chloride solution. The osmotic flow of water across the membrane from the water compartment to the sodium chloride solution compartment was monitored as by measuring the column of salt solution rising in the graduated pipet as a function of time over a period of a few hours. This experiment was conducted at 37° C with different concentrations of sodium chloride representing a series of osmotic pressures ranging from 10 to 400 atmospheres. Membrane permeability of semipermeable membrane, $k_2$, was thus measured as a function of osmotic pressure.

The single layer membranes were then removed and the bi-layer membranes were mounted in the Franz cells and oriented such that the semipermeable membrane faced the distilled water and osmosensitive membrane faced the sodium chloride solution. Permeability of the bi-layer membrane was measured as a function of osmotic pressure- Then, using the known value of $k_2$ the permeability of the osmosensitive membrane, $k_1$, was back calculated using Equation 1. The experimental apparatus and procedures and methods for calculating permeability values are detailed in US 6,245, 357. Permeability values were calculated as averages of permeability values measured between 4 hours and 9 hours from the start of the test.

The resulting experimental data were plotted in FIG. 4. The triangular symbols represent permeability of the osmosensitive membrane and the circular symbols represent the permeability of the semipermeable membrane of this example.

As can be seen in FIG. 4, the EC/HPC osmosensitive membrane exhibited a threshold permeability behavior. The permeability of the exemplary EC/HPC membrane did not increase substantially, until the osmotic pressure exerted across the membrane decreased to about 100 to 150 atm. Once the osmotic pressure exerted across the EC/HPC osmosensitive membrane decreased below about 100 atm to 150 atmospheres, however, the permeability of the membrane increased sharply. This threshold permeability phenomenon is due to the exponential nature of permeability with osmotic pressure as described in Equation 2. Likewise, as can be seen in FIG. 4, the CA/Lutrol semipermeable membrane exhibited a threshold permeability behavior. The permeability of the exemplary CA/Lutrol membrane did not increase substantially, until the osmotic pressure exerted across the membrane decreased to about 100 to 150 atm. Once the osmotic pressure exerted across the CA/Lutrol semipermeable membrane decreased below about 100 to 150 atmospheres, however, the permeability of the membrane increased sharply. This threshold permeability phenomenon is also due to the exponential nature of permeability with osmotic pressure as described in Equation 2.

Figure 5 illustrates the relative increase in permeability of the EC/HPC osmosensitive membrane compared to the relative increase in permeability of the CA/Lutrol membrane. At osmotic pressures at low osmotic pressure below the threshold osmotic pressure, the permeability of the semipermable membrane increases by a factor of about 15 while the relative increase in permeability of the osmosensitive membrane increased by more than 90 fold. Therefore, the increase in permeability of the EC/HPC osmosensitive membrane and to a much lesser extent the increase in permeability of the CA/Lutrol semipermeable membrane can be used to provide increased flexibility in achieving a desired delivery profile.

EXAMPLE 2

A candidate hydrophilic substance 46 which would swell in response to osmotic pressure for potential use in osmoresponsive membranes was screened experimentally. 15 grams of hydroxypropyl cellulose EFX were dissolved in 85 grams of 95/5 ethanol/water wt/wt. This solution was cast on a level glass plate, drawn with a Gardner knife having a fixed gap to evenly spread the solution, and allowed to dry. The resulting dried film was peeled from the plate and cut into sections 1 centimeter wide and 4 centimeters long. Individual film samples were weighed on an analytical balance and then were then individually bagged in a nylon mesh netting by heat sealing the edges. The mesh openings of the nylon bag were approximately 20 per inch. The weighed sample was then immersed in a solution of the nonionic osmoagent, sorbitol, having a known concentration of 932 mg per milliliter and maintained at a temperature of 37°C. Osmotic pressure of the solution had been measured to be 350 atmospheres. After 15 minutes, the netted film sample was removed and weighed. The wet weight was recorded. The film was then dissolved away with de-ionized water and the bag was re-immersed in the osmotic solution. The empty bag was then weighed wet. Finally, the weight gain of the film sample was calculated using Equation 6,

$$\text{Weight Gain (\%)} = [\ (W_{tw} - W_n - W_{df})/W_{df}\ ] \times 100 \qquad \text{(Eq. 6)}$$

where $W_{tw}$ represents the total wet weight of the film and bag, $W_n$ represents the weight of the wet bag, and $W_{df}$ represents the weight of the dry film. This procedure was repeated with films immersed for longer durations of time representing 35, 60, and 120 minutes. This procedure was also performed in sorbitol solutions having concentrations of 652, 466, 317, 228, 163, 98, 64, and 33 mg per milliter corresponding to fixed osmotic pressures of 170, 97, 57, 36,25,14, 8, and 4 atmospheres, respectively. Below 100 atmospheres, the films became too fragile to handle. The quantitative and qualitative results of this testing are plotted in FIG. 6.

As is illustrated in FIG. 6, swelling of the film, as represented by weight gain of the film, increased with decrease in osmotic pressure. The swelling increased steeply between about 150 atmospheres and 100 atmospheres. This experiment indentifies the threshold permeability zone between 100 and 150 atmospheres wherein the osmosensitive hydrophilic substance 48 is most responsive to changes in osmotic pressure. Above this osmotic pressure, the film is clear, glassy, hard and relatively impermeable to water. Between about 60 atmospheres and 15 atmospheres, the film hydrates to such an extent that it becomes opaque and disintegrates, and below about 15 atmospheres, the film dissolves. The data also demonstrate that the swelling of this osmoresponsive film in the presence of osmoagent sorbitol is independent of time at a give fixed osmotic pressure.

EXAMPLE 3

A candidate hydrophilic substance 46 which would swell in response to both osmotic pressure and to ionic strength for potential use in osmoresponsive membranes with osmotic core 20 formulated with an ionic osmoagent was screened experimentally. The procedures and materials detailed in EXAMPLE 2 were repeated except sorbitol was replaced with an ionic salt, sodium chloride, as the osmoagent. Sodium chloride is an electrolyte and therefore imparts ionic strength in aqueous solution. Sodium chloride concentrations of 250, 175, 125, 88, 63, 38, 25, 18, and 8 milligrams per milliliter were tested. This series of concentrations corresponds to ionic strengths of 4.28, 2.99, 2.14, 1.51, 1.08, 0.65, 0.43, 0.31, 0.14 molar and to osmotic pressures of 283, 195, 115, 78, 59,33,21,14, and 6 atmospheres, respectively. The results of this test are plotted in FIG. 7.

As is illustrated in FIG. 7, swelling of the film, as represented by weight gain of the film, increased with decrease in osmotic pressure. The swelling increased steeply between 190 atmospheres and 120 atmospheres. This experiment indentifies the threshold permeability zone wherein the osmosensitive hydrophilic substance is most responsive to changes in osmotic pressure with sodium chloride. Between about 60 atmospheres and 20 atmospheres, the film disintegrates, and below about 20 atmospheres, the film dissolves. Unlike the swelling of this osmoresponsive film in the presence of osmoagent sorbitol, the swelling of this osmoresponsive film in the presence of sodium chloride is dependent on time at a given fixed osmotic pressure. The film continues to swell and therefore become more permeable as a function of time at fixed osmotic pressure. Therefore, this screening experiment reveals a surprising finding that sodium chloride as an ion pair in water interacting with the hydrophilic substance provides a useful means of modulating the permeability of osmosensitive membranes. The osmoagent sorbitol of EXAMPLE 2 does not cause increase in swelling with time whereas the osmoagent sodium chloride does cause an increase in swelling with time at fixed osmotic pressure.

EXAMPLE 4

Characterization of the time dependency of permeability of osmosensitive membranes interacting with the ionic osmoagent, sodium chloride, was expanded. The methods and materials were the same as detailed in EXAMPLE 1 except that the individual permeability measurements were collected as a function time and plotted individually rather than plotted as averaged values. Also, measurements were collected over a period 24 hours rather than over a few hours.

FIG 8 shows the resulting three-dimensional surface plot of permeability $k_1$ as a function of osmotic pressure and time for osmoresponsive membrane 16. This plot confirms the steep increase in permeability as osmotic pressure declines below the threshold value of about 190-120 atmospheres and also reveals the increase in permeability with time at a fixed osmotic pressure which time dependency is absent in the nonionic osmoagent sorbitol.

FIG 9 shows the resulting three-dimensional surface plot of permeability $k_2$ as a function of osmotic pressure and time for semipermeable membrane. This plot reveals the less steep increase in permeability as osmotic pressure declines below the threshold value of about 190-120 atmospheres and also reveals the increase in permeability with time at a fixed osmotic pressure, which time dependency is absent in the nonionic osmoagent sorbitol.

These surface plots reveal the increase in permeability with time at fixed osmotic pressure and increase in permeability with declining osmotic pressure when the ionic osmoagent sodium chloride is coupled with the membranes of the present invention for use to control the hydration rate and expansion rate of the light push layer.

EXAMPLE 5

An exemplary dosage form according to the present invention was manufactured. The dosage form produced included nifedipine as the active agent in the active agent composition. Nifedipine is an insoluble calcium channel blocker active agent drug used to treat angina and hypertension. The drug is commercially available in immediate release capsule form or in controlled release form. Because the biological half-life of the drug is only about 2 hours, it is necessary that the drug be administered frequently in small doses in order to maintain the therapeutic level of drug in the plasma. Patients being treated using the immediate release form of the drug are required to take the medication three times each day. Patients who are prescribed the controlled release form, Procardia XL®, are able to take the medication once a day. Once a day dosing is feasible because the delivery system dispenses the daily dose of drug slowly and continuously over a prolonged 14 to 16 hour period. While the once a day dosing is preferred by patients, a smaller tablet size would be more even acceptable for patients to swallow.

The Procardia XL dosage form is available in three strengths: 30 mg, 60 mg, and 90 mg. The most commonly prescribed dose is 30 mg. This 30 mg system is manufactured with a bi-layer, round osmotic core that has a total drug loading of 12 wt%, a diameter of 0.344 inch, and a nominal weight of 247.5 mg. The laminate of the osmotic core consists of the drug layer that weighs 165 mg and a heavy push layer that weight 82.5 mg. There is no sodium chloride in the drug layer, and there is 30 wt% sodium chloride in the push layer. Due to the performance inefficiency of this system, the drug layer is formulated with 33 mg of active agent to assure that the target dose of 30 mg is delivered. Typically, about 3 mg of the active agent is trapped in the system as a non-deliverable overage after the functional period of delivery. The heavy push layer of 82.5 mg represents 1/3 of the mass of the osmotic core. This commercial product is coated with a single layer rate controlling membrane comprising 95 wt% percent cellulose acetate and 5 wt% polyethylene glycol.

The exemplary dosage nifedipine dosage form according to the present invention ("the exemplary dosage form") included a 130 mg osmotic core coated with a bi-layer membrane system. The osmotic core of the exemplary dosage form was formulated with 7 wt% sodium chloride in the drug layer and 7 wt% sodium chloride in the light push layer in order to provide the proper osmotic activity and ionic strength to activate the osmoresponsive membrane system and yield an accelerating hydration of the light push layer.

First, 57.5 grams of Polyox N80 was passed through a stainless steel mesh having 40 wires per inch. The Polyox N80 is polyoxyethylene having a number average molecular weight of approximately 200,000 grams per mole. It serves as the polymer hydrogel of the active agent composition. The sized Polyox N80 was then dry mixed with 30 grams of nifedipine that had been air jet milled to an average particle size of 5 microns. Then, 7.0 grams of the osmoagent, sodium chloride, and 5.0 grams of the tablet binder, hydroxypropyl methylcellulose E5, were sized through the mesh and thoroughly mixed with the other components until a uniform dry mixture was formed. The hydroxypropyl methycellulose had a number average molecular weight of approximately 11,300 grams per mole. Polyox and hydroxypropyl methylcellulose are available from Dow Chemical of Midland, Michigan. Then, de-natured ethyl alcohol formula 3A was added slowly to the dry mixture with stirring until a uniform damp mass was produced. This damp mass was forced through a mesh having 20 wires per inch, forming elongated pellets. The resulting extruded pellets were dried overnight in a forced air oven at 40° C. The dried extrusions were then forced again through the 20-mesh sieve, thereby breaking them up to form free-flowing granules. Finally, 0.5 grams of the tableting lubricant, magnesium stearate, was passed through a mesh with 60 wires per inch over the dried granules and tumble mixed into the blend. The resulting composition formed the active agent composition granulation.

Next, 89.5 grams of Polyox 303, 7.0 grams of the osmoagent, sodium chloride, and 3.0 grams of the hydroxypropyl methylcellulose E5 were individually passed through a 40-mesh sieve and thoroughly mixed. This grade of Polyox has a number average molecular weight of approximately 7 million and served as the osmopolymer of the light push layer. The dry mix was wet granulated using the procedure described to form the active agent composition drug layer granulation. The granules were lubricated according the same procedure with 0.5 grams of minus 60-mesh magnesium stearate. The resulting composition formed light push layer granulation.

Bilayer osmotic cores were tableted with the resulting two granulations. 100 mg of active agent composition granulation was filled into a round die cavity having a diameter of 0.250 inch and lightly tamped. Then, 30 mg of the light push layer composition was added to the cavity and was laminated to the active agent granulation by compressing with standard biconvex round tooling using a force of 1 ton. This formed a tableted bi-layer osmotic core. The resulting osmotic cores each weighed 130 mg.

The resulting batch of osmotic cores was then divided into two sublots that were subsequently coated with different rate controlling membranes. Each sublot of osmotic cores was pan coated according to the procedures detailed in EXAMPLE 1. The first sublot of osmotic cores was used to create a batch of exemplary dosage forms according to the present invention. To create these exemplary dosage forms, the osmotic cores of the first sublot were coated with the bilayer membrane system of the present invention. The bilayer membrane system was fabricated with a first osmosensitive wall having a thickness of 5.0 mils and a second semipermeable wall having a thickness of 3.0 mils. The composition of the first coat consisted of 55 wt% EC, 40 wt% HPC, 5 wt% Myrj 52S , with the total of each component equally 100 wt%. The second coated layer consisted of 75 wt% cellulose acetate and 25 wt% Lutrol. The grades of EC, HPC, Myrj, CA, and Lutrol were equivalent to those grades detailed in EXAMPLE 1. A delivery passageway was mechanically drilled through the coated layers using a 20-mil diameter bit to form a passageway connecting the active agent composition to the outside environment. The exemplary dosage forms were completed by drying the coated and drilled systems in a forced air oven at 40° C to remove residual solvents.

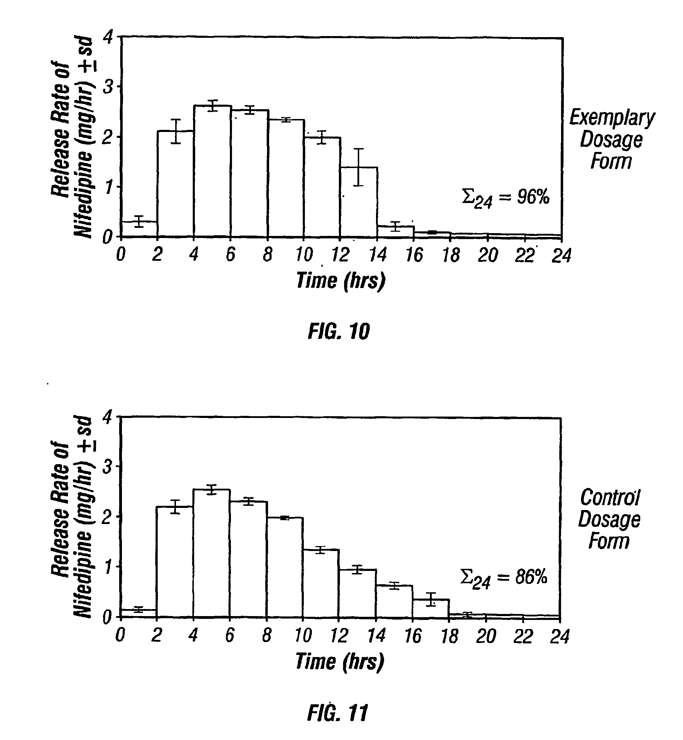

The resulting exemplary dosage forms were tested in vitro for release of the active agent. Each dosage form being tested was glued to a plastic rod and immersed in test tubes containing 45 milliliters of de-ionized water maintained at 37°C. The exemplary dosage forms were shaken vertically with a frequency 30 cycles per minute and with amplitude of 2 centimeters. After 2 hours, the exemplary dosage forms were transferred to a fresh set of water receptors and the systems were allowed to release 2 additional hours. This process was repeated until samples representing 24 hours duration were collected. The drug in each test tube was then analyzed by mixing about 40 milliters of polyethylene glycol 400 molecular weight to each receptor with stirring to dissolve the drug. Then, the samples were exposed to bright white light until the drug solutions changed from a yellow color of the drug to a colorless solution. This photo-degradation produces a colorless degradant that has a chromophore active in the ultraviolet spectrum, which is useful to assay the drug. The light-degraded samples were assayed using a spectrophotometer at a wavelength of 282 nanometers. Six of the exemplary dosage forms were tested in this experiment. The release rate of drug as a function of time is plotted in FIG. 10. The results of the test were that between hours 2 and 12, the average release rate of nifedipine was 2.3 mg per hour and the pattern was substantially zero order, or constant rate during this interval for up to about 12 hours.

The second sublot of osmotic cores was used to create a group of experimental control dosage forms. The experimental control dosage forms were created by coating the second sublot of osmotic cores with a semipermeable membrane only (without the osmosensitive membrane subcoat). The composition of this semipermeable membrane was identical to the composition of the semipermeable membrane of sublot 1 but coated to a thickness of 13.6 mils. These systems were drilled, dried, and tested for release of drug according to the same procedures used for sublot 1. The resulting release pattern is shown in FIG. 11. The average release rate of 2.3 mg per hour was maintained between hours 2 and 8, but after 8 hours, the system did not sustain this constant rate and the delivery rate declined continuously. As is easily appreciated by reference to FIG. 10 and FIG. 11, the exemplary dosage forms achieved a delivery efficiency at 24 hours of 96%, which was significantly better than the 86% delivery efficiency achieved by the experimental control dosage forms. Moreover, the release rate profile provided by the exemplary dosage forms more closely approximated a zero order release rate profile required for this drug than that achieved by the experimental control dosage forms. The exemplary dosage forms also provided a more discrete cut-off of nifedipine delivery than did the experimental control dosage forms.

The drug loading of the osmotic core of the exemplary nifedipine dosage form according to the present invention was approximately 23%, nearly double the drug loading provided by the commercial Procardia XL product. Moreover, the diameter of the exemplary dosage form measured only 0.250 inches, roughly ¼ smaller than the diameter of the comparable commercially available Procardia XL 30 mg dosage form.

This example was based on the 30 mg dose. The 90 mg commercial product is a very large, round tablet with a nominal tablet weight of 742.5 mg and a tablet diameter of 15/32 inch. Due to the delivery inefficiency of this system, it is formulated with a 10% excess drug and a heavy push layer that is 1/3 the weight of the osmotic core tablet. Some patients object to or are unable to swallow such a large round tablet. The benefit of a smaller dosage form the present invention not requiring excess drug or excess push layer is expected to provide a form of the drug at the 90 mg dose that some patients would otherwise not be willing or able to swallow.

EXAMPLE 6

Two additional sublots of bi-layer osmotic cores equivalent to those described in EXAMPLE 4 were fabricated and coated with rate controlling membranes. One sublot of osmotic cores was coated with the bi-layer membrane system of the present invention to produce a second batch of exemplary dosage forms. The bi-layer membrane system of these second exemplary dosage forms was fabricated with a first coat of 5.0 mils of the same composition as was used in EXAMPLE 4, but the semipermable membrane consisted of 2.6 mils of 70 wt% cellulose acetate blended with 30 wt% Lutrol. The second sublot of osmotic cores was used to produce a second batch of experimental control dosage forms. These second experimental control dosage forms were created by coating the osmotic cores with a single layer membrane that consisted of 3.8 mils of 90 wt% cellulose acetate blended with 10 wt% Lutrol. The grades of EC, HPC, Myrj , CA, and Lutrol were equivalent to those grades detailed in EXAMPLE 1. To provide the dosage forms with a delivery passageway, the second exemplary dosage forms and the second experimental control dosage forms were mechanically drilled through the coated layers using a 20-mil diameter bit to create a passageway connecting the active agent composition to the outside environment. The systems were dried in a forced air oven at 40° C to remove residual solvents and tested for release of drug.

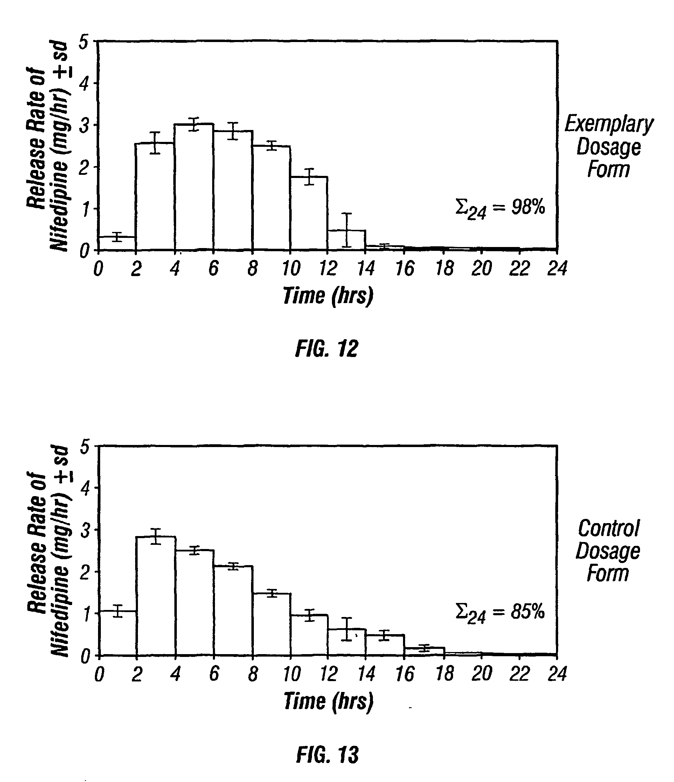

The results of the comparative tests are presented in FIG. 12 and FIG. 13. The top graph included in FIG 12, illustrates the release rate profile and delivery efficiency achieved by the second exemplary dosage forms, while the bottom graph illustrated in FIG 13 illustrates the release rate profile and delivery efficiency achieved by the second experimental control dosage forms. The second exemplary dosage forms provided a longer duration of steady state delivery and more complete delivery of nifedipine (98%) than the second experimental control dosage forms, which provided only delivered only 85% of the nifedipine in the same amount time.

Because the same osmotic cores were used in the first exemplary dosage forms and the second exemplary dosage forms, the drug loading of the osmotic core of the second exemplary dosage form was also approximately 23%, nearly double the drug loading provided by the commercial Procardia XL product. Moreover, the diameter of the second exemplary dosage was also significantly smaller than the diameter of the comparable commercially available Procardia XL 30 mg dosage form.

**Claims**

1.  A controlled release dosage form comprising:

    an osmotic core including an active agent composition and an expandable push layer, wherein the expandable push layer accounts for less than one-fourth of the osmotic core;
    a bi-layer membrane system positioned around at least a portion of the osmotic core, the bi-layer membrane system comprising a semipermeable membrane and an osmoresponsive membrane; and
    a delivery passageway.

2.  The controlled release dosage form of claim 1, wherein the osmoresponsive membrane is formulated such that the osmoresponsive membrane exhibits a threshold permeability behavior.

3.  The controlled release dosage form of claim 1, wherein the osmoresponsive membrane is formulated such that the osmosensitive membrane exhibits a permeability threshold, and the active agent composition and the expandable push layer are formulated to provide an osmotic core that exerts an osmotic pressure that is at or above the permeability threshold of the osmotic membrane.

4.  The controlled release dosage form of claim 1, wherein the active agent is selected from: hydrocodone, methylphenidate, oxybutynin, oxycodone, oxymorphone, verapamil and soluble derivatives thereof;
    hydromorphone, nifedipine, respiridone, topiramate and insoluble derivatives thereof;
    and pro-drugs, isomers and salts thereof.

5.  The controlled release dosage form of claim 1, wherein the expandable push layer accounts for less than one-fifth of the osmotic core.

6.  The controlled release dosage form of claim 1, wherein the semipermeable membrane is formed of a polymer.

7.  The controlled release dosage form of claim 1, wherein the semipermeable membrane is formed of a cellulosic polymer having a degree of substitution on an anydroglucose unit ranging from greater than 0 up to 3.

8.  The controlled release dosage form of clam 1, wherein the semipermeable membrane comprises a composition that exhibits a variable permeability, the variable permeability being responsive to changes in osmotic pressure.

9.  The controlled release dosage form of claim 1, wherein the semipermeable membrane comprises a composition that exhibits a variable permeability, wherein the permeability of the composition increases in response to decreases in osmotic pressure.

10. The controlled release dosage form of claim 1, wherein the osmoresponsive membrane comprises a hydrophobic material and a hydrophilic material.

11. The controlled release dosage form of claim 1, wherein the osmoresponsive membrane comprises an ethylcellulose and a hydroxyalkylcellulose.

12. The controlled release dosage form of claim 1, wherein the osmoresponsive membrane comprises a 40 wt% to about 99 wt% ethylcellulose and about 1 wt% to about 60% hydroxyalkyl cellulose.

13. The controlled release dosage form of claim 11, wherein the osmoresponsive membrane further includes a surfactant.

**14.** The controlled release dosage form of claim 12, wherein the osmoresponsive membrane further includes 1 wt% to 30 wt% surfactant.

**15.** The controlled release dosage form of claim 1, wherein the osmoresponsive membrane exhibits a variable permeability, wherein the controlled release dosage form is fabricated such that the variable permeability of the osmoresponsive membrane varies over time and in response to changes in osmotic pressure.

**16.** The controlled release dosage form of claim 1, wherein the osmoresponsive membrane exhibits a variable permeability, wherein the controlled release dosage form is fabricated such that osmosensitive membrane exhibits a relatively higher permeability over time and in response to decreases in osmotic pressure.

**17.** The controlled release dosage form of claim 1, wherein the osmoresponsive membrane comprises materials that result in an osmoresponsive membrane exhibiting a permeability threshold of about 100 to 150 atm.

**18.** The controlled release dosage form of claim 17, wherein the active agent composition and the expandable push layer are formulated to provide an osmotic core that exerts an initial osmotic pressure of about 100 to 150 atm, or above.

**19.** The controlled release dosage form of claim 1, wherein the osmoresponsive membrane comprises materials that result in an osmoresponsive membrane exhibiting a permeability threshold of about 120 to 190 atm.

**20.** The controlled release dosage form of claim 19, wherein the active agent composition and the expandable push layer are formulated to provide an osmotic core that exerts an initial osmotic pressure of about 120 to 190 atm, or above.

**21.** The controlled release dosage form of claim 1, wherein:

the osmoresponsive membrane exhibits a variable permeability, with the osmoresponsive membrane being formulated such that the permeability of the osmoresponsive membrane increases at an exponential rate as osmotic pressure across the membrane decreases below a threshold osmotic pressure; and
an osmotic core comprising an active agent composition and an expandable push layer, wherein the active agent composition and the expandable push layer comprise materials that exert an initial osmotic pressure across the osmoresponsive membrane that is at or above the threshold osmotic pressure.

**22.** The controlled release dosage form of claim 21, wherein the semipermeable membrane is formed of a polymer.

**23.** The controlled release dosage form of claim 21, wherein the semipermeable membrane is formed of a cellulosic polymer having a degree of substitution on an anydroglucose unit ranging from greater than 0 up to 3.

**24.** The controlled release dosageform of clam 21, wherein the semipermeable membrane comprises a composition that exhibits a variable permeability, the variable permeability being responsive to changes in osmotic pressure.

**25.** The controlled release dosage form of claim 21, wherein the semipermeable membrane comprises a composition that exhibits a variable permeability, wherein the permeability of the composition increases in response to decreases in osmotic pressure,

**26.** The controlled release dosage form of claim 21, wherein the expandable push layer accounts for less than one-fifth of the osmotic core.

**Patentansprüche**

**1.** Arzneiform zur kontrollierten Freisetzung, welche:

einen osmotischen Kern, der eine Wirkstoffzusammensetzung und eine expandierbare Schubschicht einschließt, wobei die expandierbare Schubschicht weniger als ein Viertel des osmotischen Kerns ausmacht;
ein Zwei-Schicht-Membransystem, das um wenigstens einen Teil des osmotischen Kerns herum angeordnet ist, wobei das Zwei-Schicht-Membransystem eine semipermeable Membran und eine osmoresponsive Membran umfasst; und
einen Abgabedurchlass umfasst.

2. Arzneiform zur kontrollierten Freisetzung nach Anspruch 1, **dadurch gekennzeichnet, dass** die osmoresponsive Membran so formuliert ist, dass die osmoresponsive Membran ein Schwellenpermeabilitätsverhalten zeigt.

3. Arzneiform zur kontrollierten Freisetzung nach Anspruch 1, **dadurch gekennzeichnet, dass** die osmoresponsive Membran so formuliert ist, dass die osmosensitive Membran einen Permeabilitätsschwellenwert zeigt, und die Wirkstoffzusammensetzung und die expandierbare Schubschicht formuliert sind, um einen osmotischen Kern bereitzustellen, der einen osmotischen Druck ausübt, der bei oder oberhalb des Permeabilitätsschwellenwertes der osmotischen Membran liegt.

4. Arzneiform zur kontrollierten Freisetzung nach Anspruch 1, **dadurch gekennzeichnet, dass** der Wirkstoff ausgewählt ist aus: Hydrocodon, Methylphenidat, Oxybutynin, Oxycodon, Oxymorphon, Verapamil und löslichen Derivaten derselben;
Hydromorphon, Nifedipin, Respiridon, Topiramat und unlöslichen Derivaten derselben;
und Prodrugs, Isomeren und Salzen davon.

5. Arzneiform zur kontrollierten Freisetzung nach Anspruch 1, **dadurch gekennzeichnet, dass** die expandierbare Schubschicht weniger als ein Fünftel des osmotischen Kerns ausmacht.

6. Arzneiform zur kontrollierten Freisetzung nach Anspruch 1, **dadurch gekennzeichnet, dass** die semipermeable Membran aus einem Polymer gebildet ist.

7. Arzneiform zur kontrollierten Freisetzung nach Anspruch 1, **dadurch gekennzeichnet, dass** die semipermeable Membran aus einem Cellulosepolymer mit einem Substitutionsgrad auf einer Anhydroglucose-Einheit im Bereich von mehr als 0 bis zu 3 gebildet ist.

8. Arzneiform zur kontrollierten Freisetzung nach Anspruch 1, **dadurch gekennzeichnet, dass** die semipermeable Membran eine Zusammensetzung umfasst, die eine variable Permeabilität zeigt, wobei die variable Permeabilität auf Veränderungen im osmotischen Druck anspricht.

9. Arzneiform zur kontrollierten Freisetzung nach Anspruch 1, **dadurch gekennzeichnet, dass** die semipermeable Membran eine Zusammensetzung umfasst, die eine variable Permeabilität zeigt, wobei die Permeabilität der Zusammensetzung in Reaktion auf Verringerungen im osmotischen Druck ansteigt.

10. Arzneiform zur kontrollierten Freisetzung nach Anspruch 1, **dadurch gekennzeichnet, dass** die osmoresponsive Membran ein hydrophobes Material und ein hydrophiles Material umfasst.

11. Arzneiform zur kontrollierten Freisetzung nach Anspruch 1, **dadurch gekennzeichnet, dass** die osmoresponsive Membran eine Ethylcellulose und eine Hydroxyalkylcellulose umfasst.

12. Arzneiform zur kontrollierten Freisetzung nach Anspruch 1, **dadurch gekennzeichnet, dass** die osmoresponsive Membran etwa 40 Gew.-% bis etwa 99 Gew.-% Ethylcellulose und etwa 1 Gew.-% bis etwa 60 Gew.-% Hydroxyalkylcellulose umfasst.

13. Arzneiform zur kontrollierten Freisetzung nach Anspruch 11, **dadurch gekennzeichnet, dass** die osmoresponsive Membran weiter ein Tensid einschließt.

14. Arzneiform zur kontrollierten Freisetzung nach Anspruch 12, **dadurch gekennzeichnet, dass** die osmoresponsive Membran weiter 1 Gew.-% bis 30 Gew.-% Tensid einschließt.

15. Arzneiform zur kontrollierten Freisetzung nach Anspruch 1, **dadurch gekennzeichnet, dass** die osmoresponsive Membran eine variable Permeabilität zeigt, wobei die Arzneiform zur kontrollierten Freisetzung so hergestellt wird, dass die variable Permeabilität der osmoresponsiven Membran über die Zeit und in Reaktion auf Veränderungen im osmotischen Druck variiert.

16. Arzneiform zur kontrollierten Freisetzung nach Anspruch 1, **dadurch gekennzeichnet, dass** die osmoresponsive Membran eine variable Permeabilität zeigt, wobei die Arzneiform zur kontrollierten Freisetzung so hergestellt wird, dass die osmosensitive Membran eine relativ höhere Permeabilität über die Zeit und in Reaktion auf Verringerungen im osmotischen Druck zeigt.

**17.** Arzneiform zur kontrollierten Freisetzung nach Anspruch 1, **dadurch gekennzeichnet, dass** die osmoresponsive Membran Materialien umfasst, die zu einer osmoresponsiven Membran führen, die einen Permeabilitätsschwellenwert von etwa 100 bis 150 atm zeigt.

**18.** Arzneiform zur kontrollierten Freisetzung nach Anspruch 17, **dadurch gekennzeichnet, dass** die Wirkstoffzusammensetzung und die expandierbare Schubschicht formuliert sind, um einen osmotischen Kern bereitzustellen, der einen anfänglichen osmotischen Druck von etwa 100 bis 150 atm oder darüber ausübt.

**19.** Arzneiform zur kontrollierten Freisetzung nach Anspruch 1, **dadurch gekennzeichnet, dass** die osmoresponsive Membran Materialien umfasst, die zu einer osmoresponsiven Membran führen, die einen Permeabilitätsschwellenwert von etwa 120 bis 190 atm zeigt.

**20.** Arzneiform zur kontrollierten Freisetzung nach Anspruch 19, **dadurch gekennzeichnet**, das die Zusammensetzung und die expandierbare Schubschicht formuliert sind, um einen osmotischen Kern bereitzustellen, der einen anfänglichen osmotischen Druck von etwa 120 bis 190 atm oder darüber ausübt.

**21.** Arzneiform zur kontrollierten Freisetzung nach Anspruch 1, **dadurch gekennzeichnet, dass**:

die osmoresponsive Membran eine variable Permeabilität zeigt, wobei die osmoresponsive Membran so formuliert ist, dass die Permeabilität der osmoresponsiven Membran mit einer exponentiellen Rate ansteigt, wenn der osmotische Druck über die Membran unter einen osmotischen Schwellendruck absinkt; und mit einem osmotischen Kern, der eine Wirkstoffzusammensetzung und eine expandierbare Schubschicht umfasst, wobei die Wirkstoffzusammensetzung und die expandierbare Schubschicht Materalien umfassen, die einen anfänglichen osmotischen Druck über die osmoresponsive Membran ausüben, der bei oder oberhalb des osmotischen Schwellendrucks liegt.

**22.** Arzneiform zur kontrollierten Freisetzung nach Anspruch 21, **dadurch gekennzeichnet, dass** die semipermeable Membran aus einem Polymer gebildet ist.

**23.** Arzneiform zur kontrollierten Freisetzung nach Anspruch 21, **dadurch gekennzeichnet, dass** die semipermeable Membran aus einem Cellulosepolymer mit einem Substitutionsgrad auf einer Anhydroglucose-Einheit in einem Bereich von mehr als 0 bis zu 3 gebildet ist.

**24.** Arzneiform zur kontrollierten Freisetzung nach Anspruch 21, **dadurch gekennzeichnet, dass** die semipermeable Membran eine Zusammensetzung umfasst, die eine variable Permeabilität zeigt, wobei die variable Permeabilität auf Veränderungen im osmotischen Druck anspricht.

**25.** Arzneiform zur kontrollierten Freisetzung nach Anspruch 21, **dadurch gekennzeichnet, dass** die semipermeable Membran eine Zusammensetzung umfasst, die eine variable Permeabilität zeigt, wobei die Permeabilität der Zusammensetzung in Reaktion auf Verringerungen im osmotischen Druck ansteigt.

**26.** Arzneiform zur kontrollierten Freisetzung nach Anspruch 21, **dadurch gekennzeichnet, dass** die expandierbare Schubschicht weniger als ein Fünftel des osmotischen Kerns ausmacht.

**Revendications**

**1.** Forme galénique à libération contrôlée comprenant :

un noyau osmotique incluant une composition d'agent actif et une couche de poussée expansible, où la couche de poussée expansible représente moins d'un quart du noyau osmotique ; un système de membranes bicouche positionné autour d'au moins d'une partie du noyau osmotique, le système de membranes bicouche comprenant une membrane semi-perméable et une membrane osmosensible ; et un passage de distribution.

**2.** Forme galénique à libération contrôlée selon la revendication 1 où la membrane osmosensible est formulée de telle manière que la membrane osmosensible présente un comportement de perméabilité à seuil.

**3.** Forme galénique à libération contrôlée selon la revendication 1 où la membrane osmosensible est formulée de telle manière que la membrane osmosensible présente un seuil de perméabilité, et la composition d'agent actif et la couche de poussée expansible sont formulées pour produire un noyau osmotique qui exerce une pression osmotique qui est à ou au-delà du seuil de perméabilité de la membrane osmotique.

**4.** Forme galénique à libération contrôlée selon la revendication 1 où l'agent actif est choisi parmi : hydrocodone, méthylphénidate, oxybutynine, oxycodone, oxymorphone, verapamil et leurs dérivés solubles ; hydromorphone, nifédipine, respiridone, topiramate et leurs dérivés insolubles ; et leurs pro-médicaments, isomères et sels.

**5.** Forme galénique à libération contrôlée selon la revendication 1 où la couche de poussée expansible représente moins d'un cinquième du noyau osmotique.

**6.** Forme galénique à libération contrôlée selon la revendication 1 où la membrane semi-perméable est formée d'un polymère.

**7.** Forme galénique à libération contrôlée selon la revendication 1 où la membrane semi-perméable est formée d'un polymère cellulosique ayant un degré de substitution sur une unité d'anhydroglucose allant de plus de 0 jusqu'à 3.

**8.** Forme galénique à libération contrôlée selon la revendication 1 où la membrane semi-perméable comprend une composition qui présente une perméabilité variable, la perméabilité variable étant sensible à des changements de pression osmotique.

**9.** Forme galénique à libération contrôlée selon la revendication 1 où la membrane semi-perméable comprend une composition qui présente une perméabilité variable, où la perméabilité de la composition augmente en réponse à des diminutions de pression osmotique.

**10.** Forme galénique à libération contrôlée selon la revendication 1 où la membrane osmosensible comprend une substance hydrophobe et une substance hydrophile.

**11.** Forme galénique à libération contrôlée selon la revendication 1 où la membrane osmosensible comprend une éthylcellulose et une hydroxyalkylcellulose.

**12.** Forme galénique à libération contrôlée selon la revendication 1 où la membrane osmosensible comprend 40 % en masse à environ 99 % en masse d'éthylcellulose et environ 1 % en masse à environ 60 % d'hydroxyalkylcellulose.

**13.** Forme galénique à libération contrôlée selon la revendication 11 où la membrane osmosensible inclut en outre un tensioactif.

**14.** Forme galénique à libération contrôlée selon la revendication 12 où la membrane osmosensible inclut en outre 1 % en masse à 30 % en masse de tensioactif.

**15.** Forme galénique à libération contrôlée selon la revendication 1 où la membrane osmosensible présente une perméabilité variable, où la forme galénique à libération contrôlée est fabriquée de telle manière que la perméabilité variable de la membrane osmosensible varie au cours du temps et en réponse à des changements de pression osmotique.

**16.** Forme galénique à libération contrôlée selon la revendication 1 où la membrane osmosensible présente une perméabilité variable, où la forme galénique à libération contrôlée est fabriquée de telle manière que la membrane osmosensible présente une perméabilité relativement plus grande au cours du temps et en réponse à des diminutions de pression osmotique.

**17.** Forme galénique à libération contrôlée selon la revendication 1 où la membrane osmosensible comprend des substances qui conduisent à une membrane osmosensible présentant un seuil de perméabilité d'environ 100 à 150 atm.

**18.** Forme galénique à libération contrôlée selon la revendication 17 où la composition d'agent actif et la couche de poussée expansible sont formulées pour produire un noyau osmotique qui exerce une pression osmotique initiale d'environ 100 à 150 atm, ou plus.

**19.** Forme galénique à libération contrôlée selon la revendication 1 où la membrane osmosensible comprend des substances qui conduisent à une membrane osmosensible présentant un seuil de perméabilité d'environ 120 à 190 atm.

**20.** Forme galénique à libération contrôlée selon la revendication 19 où la composition d'agent actif et la couche de poussée expansible sont formulées pour produire un noyau osmotique qui exerce une pression osmotique initiale d'environ 120 à 190 atm, ou plus.

**21.** Forme galénique à libération contrôlée selon la revendication 1 où :

la membrane osmosensible présente une perméabilité variable, la membrane osmosensible étant formulée de telle manière que la perméabilité de la membrane osmosensible augmente de manière exponentielle quand la pression osmotique à travers la membrane diminue en dessous d'une pression osmotique de seuil ; et
un noyau osmotique comprenant une composition d'agent actif et une couche de poussée expansible, où la composition d'agent actif et la couche de poussée expansible comprennent des substances qui exercent une pression osmotique initiale à travers la membrane osmosensible qui est à ou au-delà de la pression osmotique de seuil.

**22.** Forme galénique à libération contrôlée selon la revendication 21 où la membrane semi-perméable est formée d'un polymère.

**23.** Forme galénique à libération contrôlée selon la revendication 21 où la membrane semi-perméable est formée d'un polymère cellulosique ayant un degré de substitution sur une unité d'anhydroglucose allant de plus de 0 jusqu'à 3.

**24.** Forme galénique à libération contrôlée selon la revendication 21 où la membrane semi-perméable comprend une composition qui présente une perméabilité variable, la perméabilité variable étant sensible à des changements de pression osmotique.

**25.** Forme galénique à libération contrôlée selon la revendication 21 où la membrane semi-perméable comprend une composition qui présente une perméabilité variable, où la perméabilité de la composition augmente en réponse à des diminutions de pression osmotique.

**26.** Forme galénique à libération contrôlée selon la revendication 21 où la couche de poussée expansible représente moins d'un cinquième du noyau osmotique.

FIG. 1

FIG. 2

FIG. 3

FIG. 4

FIG. 5

**FIG. 6**

**FIG. 7**

FIG. 8

FIG. 9

**FIG. 10**

**FIG. 11**

FIG. 12

FIG. 13

**REFERENCES CITED IN THE DESCRIPTION**

**Patent documents cited in the description**

- US 3845770 A
- US 3916899 A
- US 4008719 A
- US 4014334 A
- US 4058122 A
- US 4116241 A
- US 4160452 A

- US 5160744 A
- US 6245357 B
- US 4063064 A, Saunders
- US 4088864 A, Theeuwes
- US 4200098 A
- US 4285987 A

**Non-patent literature cited in the description**

- Remington's Pharmaceutical Sciences. Mack Publishing Co, 1990